# FASCICULE DE BREVET EUROPEEN

(11) **EP 2 427 497 B1**
(45) Date de publication et mention de la délivrance du brevet: **07.12.2016**
(21) Numéro de dépôt: 10727776.6
(22) Date de dépôt: 07.05.2010
(51) Int. Cl.: C07K 16/42

(54) **UTILISATION D'IMMUNOGLOBULINES IGG1 ET/OU DE LIGANDS DU RÉCEPTEUR CD32 POUR LE TRAITEMENT DE MALADIES ET MANIFESTATIONS INFLAMMATOIRES PAR VOIE MUCOSALE**
VERWENDUNG VON IGG1-IMMUNOGLOBULINEN UND/ODER LIGANDEN DES CD32-REZEPTORS ZUR BEHANDLUNG ENTZÜNDLICHER KRANKHEITEN UND EREIGNISSE ÜBER DIE SCHLEIMHAUT
USE OF IGG1 IMMUNOGLOBULINS AND/OR LIGANDS OF THE CD32 RECEPTOR FOR TREATING INFLAMMATORY DISEASES AND INCIDENTS VIA THE MUCOSA

(30) Priorité: 07.05.2009 FR 0953057
(43) Date de publication de la demande: 14.03.2012
(73) Titulaire: STALLERGENES, 92160 Antony (FR)
(72) Inventeur: BATARD, Thierry, F-78000 Versailles (FR); MOINGEON, Philippe, F-91370 Verrieres Le Buisson (FR); MASCARELL, Laurent, F-75020 Paris (FR); ZIMMER, Aline, F-91300 Massy (FR); NONY, Emmanuel, F-92160 Antony (FR)
(74) Mandataire: Lavoix
(86) Numéro de dépôt international: PCT/FR2010/050894
(87) Numéro de publication internationale: WO 2010/128265

(56) Documents cités:
- WO-A-00/50460
- WO-A-2008/099188
- WO-A1-2010/033736
- WO-A2-02/088317
- WO-A2-03/015817
- WO-A2-03/028668
- WO-A2-03/041731
- WO-A2-2004/041867
- WO-A2-2006/122257
- WO-A2-2008/086395
- US-A1- 2008 206 246
- WIGGINTON S J ET AL: "An immunoglobulin E-reactive chimeric human immunoglobulin G1 anti-idiotype inhibits basophil degranulation through cross-linking of Fc epsilon RI with Fc gamma RIIb", CLINICAL AND EXPERIMENTAL ALLERGY, vol. 38, no. 2, février 2008 (2008-02), pages 313-319, XP002555134, ISSN: 0954-7894
- RUDOLF M P ET AL: "Effect of anti-IgE antibodies on Fc epsilonRI-bound IgE.", JOURNAL OF IMMUNOLOGY (BALTIMORE, MD. : 1950) 15 DEC 1996, vol. 157, no. 12, 15 décembre 1996 (1996-12-15), pages 5646-5652, XP002555135, ISSN: 0022-1767
- ZHOU JOSEPH S ET AL: "Mast cell deficiency in Kit(W-sh) mice does not impair antibody-mediated arthritis", JOURNAL OF EXPERIMENTAL MEDICINE, vol. 204, no. 12, novembre 2007 (2007-11), pages 2797-2802, XP002555414, ISSN: 0022-1007 cité dans la demande
- SAXON A ET AL: "Accentuate the negative, eliminate the positive: Engineering allergy therapeutics to block allergic reactivity through negative signaling", JOURNAL OF ALLERGY AND CLINICAL IMMUNOLOGY, MOSBY, INC, US, vol. 121, no. 2, 1 février 2008 (2008-02-01), pages 320-325, XP002564717, ISSN: 0091-6749, DOI: DOI:10.1016/J.JACI.2007.10.017 [extrait le 2007-12-20]
- SEHRA SARITA ET AL: "Airway IgG counteracts specific and bystander allergen-triggered pulmonary inflammation by a mechanism dependent on FcgammaR and IFN-gamma.", JOURNAL OF IMMUNOLOGY, vol. 171, no. 4, 15 août 2003 (2003-08-15) , pages 2080-2089, XP002624287, ISSN: 0022-1767
- RAVIRAJAN C T ET AL: "Effect of neutralizing antibodies to IL-10 and C5 on the renal damage caused by a pathogenic human anti-dsDNA antibody", RHEUMATOLOGY (OXFORD), vol. 43, no. 4, avril 2004 (2004-04), pages 442-447, XP002624288, ISSN: 1462-0324
- RABINOVITCH N ET AL: "The role of immunoglobulin therapy in allergic diseases", ALLERGY (COPENHAGEN), vol. 54, no. 7, juillet 1999 (1999-07), pages 662-668, XP002624289, ISSN: 0105-4538
- DE GROOT ANNE S ET AL: "Activation of natural regulatory T cells by IgG Fc-derived peptide "Tregitopes"", BLOOD, vol. 112, no. 8, octobre 2008 (2008-10), pages 3303-3311, XP002624290, ISSN: 0006-4971

## Description

La présente invention concerne l'utilisation d'anticorps anti-IgE de type IgG1 ayant la capacité de lier les IgE à la surface des mastocytes ou basophiles sans dissocier lesdites IgE de leurs récepteurs et de ponter des IgE fixées aux récepteurs à la surface d'un mastocyte ou basophile, pour le traitement des hypersensibilités de type I.

Les allergies sont des réactions d'hypersensibilité anormales, inadaptées et excessives de l'organisme consécutives à un contact avec un élément, le plus souvent extérieur, l'allergène. Ces réactions sont classées en quatre groupes en fonction des mécanismes de ces réactions, aboutissant aux symptômes allergiques. L'allergie immédiate, ou hypersensibilité de type 1, est caractérisée par la libération par les mastocytes et les basophiles de médiateurs pro-inflammatoires dont l'histamine, des cytokines pro-inflammatoires et des leucotriènes (Môbs et al. (2008) Int. Arch. Allergy Immunol. 147 : 171-178) suite à une stimulation induite par le pontage d'IgE à la surface de ces cellules.

L'apparition d'une hypersensibilité de type 1 est consécutive à une série d'événements impliquant différents acteurs du système immunitaire. Un allergène va tout d'abord être pris en charge par des cellules présentatrices de l'antigène et des lymphocytes B spécifiques de l'allergène. Des fragments de cet allergène vont ensuite être présentés via le complexe majeur d'histocompatibilité, ou CMH, à des lymphocytes T aboutissant à l'activation des lymphocytes T spécifiques de l'allergène et à la sécrétion d'IL-4 par ceux-ci. L'activation de lymphocytes B par ces lympocytes T en présence d'IL-4 et de l'allergène va induire la différenciation de ces lymphocytes B en plasmocytes sécréteur d'IgE spécifiques de l'allergène. Les IgE ainsi produites vont se fixer par leur extrémité Fc à la surface des mastocytes et basophiles. L'allergène, lors d'un second contact, va ponter ces IgE et induire la libération de substances pro-inflammatoires par les basophiles et mastocytes, notamment via un phénomène de dégranulation (Bush et al. (2004) Treat. Respir. Med. 3 : 45-57 ; Strunck et al. (2006) N. Engl. J. Med. 354 : 2689-2695). Ces substances sont responsables des symptômes de l'allergie tels que l'asthme, la rhinite ou la conjonctivite.

Les IgE sont connues pour être capables de se fixer par leur partie Fc à deux types de récepteurs, le récepteur de haute affinité RFcεl et le récepteur de basse affinité RFcεII, ou CD23 (Klubal et al. (1997) J. Invest. Dermatol. 108 : 336-342, Dierks et al. (1993) J. Immunol. 150 : 2372-2382), les mastocytes et basophiles exprimant fortement le RFcεI.

L'utilisation de différentes immunoglobulines a été envisagée pour le traitement des maladies inflammatoires et d'allergies.

Ainsi, Nimmerjahn et Ravetch ont rapporté que des Ig intraveineuses (IVIG) administrées à hautes doses permettaient de traiter des maladies auto-immunes et inflammatoires (Nimmerjahn et Ravetch (2007) JEM 204:11-15**).** D'après les auteurs, l'effet anti-inflammatoire observé avec les IVIG serait lié à une population d'IgG portant des acides sialiques au niveau des glycanes N-liés au résidu Asn297 de la région Fc de ces IgG. Plus précisément, cette activité anti-inflammatoire est dépendante de la sialylation par des liaisons 2,6 de l'avant-dernier galactose de la chaîne de N-glycosylations liée au résidu Asn297 (Anthony et al. (2008) Science 320:373-376). Dans cette dernière publication, il est proposé d'administrer les IVIG par voie intraveineuse pour traiter des maladies inflammatoires.

La demande internationale WO 2008/057634 propose quant à elle d'utiliser un polypeptide comprenant au moins une région Fc d'IgG ayant un niveau de sialylation supérieur à celui d'une préparation d'anticorps non purifiée, pour traiter une maladie inflammatoire. Dans ce type de stratégie, la sialylation des IgG est montrée comme étant particulièrement importante pour obtenir une meilleure efficacité dans le traitement de la maladie inflammatoire.

La demande internationale WO 2009/079382 décrit elle aussi l'utilisation d'un polypeptide comprenant au moins une région Fc d'IgG ayant un niveau de sialylation supérieur à celui d'une préparation d'anticorps non purifiée pour le traitement de maladies inflammatoires. Là encore, la sialylation des IgG est nécessaire pour observer une activité anti-inflammatoire. Il a en effet été défini trois états distincts des IgG selon le niveau de sialylation de leur région Fc (Anthony et al. (2008) Science 320:373-376). Dans son état désialylé, les IgG confèreraient une activité cytotoxique par liaison à des récepteurs de Fc activateurs. Les IgG perdraient leur capacité cytotoxique par sialylation du glycane lié à Fc, convertissant ainsi les IgG en un état non-réactif, non-inflammatoire en réduisant la liaison au récepteur FcR. Enfin, les IgG deviendraient anti-inflammatoires par liaison de l'acide sialique 2,6 au récepteur correspondant. Il a été plus précisément montré que l'effet anti-inflammatoire de ces IVIG sous forme sialylée était dû à leur liaison au récepteur SIGN-R1, correspondant au récepteur humain DC-SIGN (Anthony et al. (2008) Proc. Natl. Acad. Science 105:19571-19578). Le récepteur SIGN-R1 est localisé, chez la souris, sur les macrophages de la zone marginale de la rate, tandis que son homologue humain DC-SIGN est présent dans les cellules dendritiques.

Pour obtenir un effet anti-inflammatoire optimal, il apparaît donc nécessaire que les récepteurs SIGN-R1/DC-SIGN soient directement accessibles aux immunoglobulines administrées. Or les présents inventeurs ont montré que ces récepteurs ne se trouvaient, au niveau de la langue, que dans le tissu musculaire, loin du site d'administration lors d'une administration par voie sublinguale. Par conséquent, l'utilisation des IVIG n'est pas appropriée pour le traitement de maladies inflammatoires par voie mucosale.

Enfin, la demande internationale WO 2003/041731 décrit l'utilisation pour le traitement de l'allergie d'un composé comprenant un domaine Fc d'IgG, spécifique au récepteur FCγ, et un domaine de fixation à un allergène.

De manière surprenante, les inventeurs ont observé une amélioration des symptômes d'asthme allergique en administrant par voie sublinguale des anticorps de type IgG₁, sous forme sialylée ou non, et sans que cette amélioration soit liée à un idiotype particulier. Ils ont en parallèle mis en évidence que les tissus de gencives humaines comprennent une proportion plus importante de récepteurs CD32 que de récepteurs activateurs CD16, en particulier au niveau du chorion papillaire. Sans vouloir être lié par un mécanisme d'action, l'effet anti-inflammatoire observé avec les anticorps de type IgG₁ (dont certains ne sont pas sialylés) administrés par voie sublinguale pourrait donc être médié par leur liaison aux récepteurs CD32.

Par ailleurs, une stratégie de thérapie de l'allergie décrite dans l'art antérieur consiste à cibler spécifiquement les IgE.

La déplétion des lymphocytes B sécréteurs d'IgE a ainsi été envisagée. Dans la demande de brevet WO2008116149, des anticorps anti-IgE capables de reconnaître des épitopes particuliers des IgE de surface des lymphocytes B ont été mis au point. Ces anticorps vont entraîner le pontage des IgE de surface et induire, en absence d'autres signaux, l'apoptose des lymphocytes B. Ces anticorps anti-IgE ont comme autre particularité de ne pas reconnaître les IgE à la surface des basophiles et des mastocytes, ceci afin d'éviter le pontage de ces anticorps à la surface de ces cellules, pontage qui entraînerait la libération de substances pro-inflammatoires.

Une alternative consistant à inhiber les lymphocytes B sécréteurs d'IgE en utilisant des molécules capables de lier à la fois les IgE de surface et CD32B est décrite dans WO2010033736. Des anticorps anti-IgE mutés pour augmenter l'affinité de la région Fc pour CD32B sont en particulier utilisées.

D'autres recherches ont également tenté d'éviter les manifestations cliniques de l'allergie en empêchant la fixation des IgE à la surface des basophiles et des mastocytes.

Ainsi, un anticorps anti-IgE humaine de type IgG1κ humanisé à 95% a été développé et utilisé en thérapie sous le nom d'Omalizumab, commercialisé sous la marque Xolair (Genentech/Novartis). Cet anticorps a été produit de façon à présenter deux caractéristiques essentielles. Tout d'abord il inhibe la liaison des IgE aux récepteurs RFcεl qui se trouvent à la surface des mastocytes et des basophiles, en se liant à un épitope de la molécule d'IgE circulante qui est impliqué dans cette liaison (Presta et al. (1993) J. Immunol. 151 : 2623-2632). Cet anticorps est ensuite, et par conséquent, incapable de reconnaître les anticorps IgE fixés sur les basophiles ou les mastocytes, le site de fixation étant masqué. Cette dernière caractéristique semblait essentielle pour éviter l'activation de ces cellules qui aurait lieu si l'anti-IgE était capable de reconnaître les IgE de surface et par conséquent de les ponter, à la façon d'un allergène.

L'utilisation d'un tel anticorps a permis de diminuer l'intensité des symptômes allergiques en séquestrant et entraînant l'élimination des IgE circulantes, ce qui, par un rétro-contrôle, induit la baisse du nombre de récepteurs RFcεl sur les mastocytes des basophiles (Bush et al. (2004) Treat. Respir. Med. 3 : 45-57, Saini et al. (1999) J. Immunol. 162 : 5624-5630). L'indication d'Omalizumab reste cependant limitée au traitement des adolescents (de 12 ans ou plus) et des adultes souffrant d'un asthme persistant modéré à sévère, qui présentent également un test cutané positif ou une réactivité in vitro à un aéro-allergène perannuel, et chez qui un traitement par corticoïdes inhalés ne permet pas de contrôler cet asthme (Bang et al. (2004) BioDrugs 18 : 415-418). Cette limitation dans l'utilisation est en particulier due à un certain nombre d'effets secondaires et de défauts de l'Omalizumab, ainsi qu'au manque d'information sur l'effet de l'Omalizumab à long terme. Parmi les effets secondaires on peut citer en particulier des réactions locales au site d'injection (Omalizumab: new drug (2007) Prescrire Int. 16 : 179-182), la réapparition d'une polypose nasale (Tonnel et al. (2006) N. Engl. J. Med. 355 : 1282), une insuffisance surrénale subaiguë (Tonnel et al. (2006) N. Engl. J. Med. 355:1282), et l'induction possible d'un syndrome de Churg et Strauss (Winchester et al. (2006) N. Engl. J. Med. 355 : 1281-1282). Des réactions d'anaphylaxie sont également observées (Omalizumab: new drug (2007) Prescrire Int. 16 : 179-182), de sorte que le produit doit obligatoirement être administré sous surveillance médicale stricte. En outre, le patient doit voir un médecin toutes les deux à quatre semaines pour une à trois injections sous-cutanées du produit. Le traitement doit théoriquement être pris à vie, dans la mesure où les effets conduisant à l'amélioration des symptômes (baisse des IgE circulantes et baisse du nombre des récepteurs à la surface des basophiles) sont réversibles lors de l'arrêt du traitement (Saini et al. (1999) J. Immunol. 162 : 5624-5630).

Il est également envisageable que l'élimination de plus de 95% des IgE circulantes devienne problématique dans la mesure où les IgE peuvent jouer un rôle dans la lutte contre les parasitoses (Cooper et al. (2008) Allergy 63 : 409-417) et/ou contre les cancers (Gould et al. (1999) Eur. J. Immunol. 29 : 3527-3537, Karagiannis et al. (2003) Eur. J. Immunol. 33: 1030-1040, Karagiannis et al. (2007) J. Immunol. 179: 2832-2843, Karagiannis et al. (2008) Cancer Immunol. Immunother. 57 : 247-263). De fait, des études cliniques ont montré que les patients traités par Omalizumab voient augmenter leurs probabilités de contracter des cancers de divers types (0,5%), en comparaison avec des patients sous placebo (0,2%).

Enfin, le traitement par Omalizumab peut s'avérer onéreux.

Afin de réduire les quantités d'anticorps anti-IgE administrées pendant le traitement, des anticorps améliorés dirigés contre les IgE ayant une très forte affinité pour celles-ci ont été décrits. Ainsi la demande de brevet EP2000481 a décrit des anticorps anti-IgE bloquant la liaison des IgE à leur récepteur de haute affinité RFcεl pour une utilisation en thérapie ainsi que pour le diagnostic de l'allergie.

Dans la demande WO2008123999, les anticorps anti-IgE utilisés sont des anticorps humains, ce qui permet d'éviter le risque d'une réaction immunitaire dirigée contre des anticorps murins humanisés.

La demande EP0550020 décrit quant à elle des anticorps capables de reconnaître les IgE fixées à la surface des mastocytes et basophiles et de les dissocier de leurs récepteurs à la surface des ces cellules. Ainsi, ces anticorps n'induisent pas le pontage des IgE et donc la libération de médiateurs pro-inflammatoires.

D'autres anticorps capables de lier les IgE fixées à la surface des mastocytes et basophiles sans les ponter sont décrits dans la demande WO0050460.

L'ensemble de ces études relatives aux anticorps anti-IgE comme vecteurs de traitement des allergies immédiates s'accorde sur le fait que de tels anticorps anti-IgE doivent nécessairement être incapables de ponter les IgE à la surface des mastocytes et basophiles, afin d'éviter la libération de médiateurs pro-inflammatoires induite par ce pontage. Par ailleurs, certaines de ces études, voire toutes, aboutissent à l'élimination de l'ensemble des IgE circulantes ce qui, comme mentionné précédemment, peut entraîner des effets secondaires gênants.

Les méthodes actuelles de traitement des allergies à l'aide d'anticorps anti-IgE sont donc insatisfaisantes compte tenu des désavantages mentionnés ci-dessus.

Les inventeurs ont désormais montré qu'une immunoglobuline de type IgG₁, notamment anti-IgE, et en particulier une IgG1 anti-IgE ayant la capacité de ponter les IgE à la surface des mastocytes et des basophiles, induit de façon surprenante une amélioration des symptômes d'asthme allergique quand elle est administrée par voie sublinguale.

### Définitions

Les termes "anticorps" et "immunoglobuline" ont la même signification et sont utilisés indifféremment dans la présente demande. Un anticorps correspond à une immunoglobuline ainsi qu'aux portions immunologiquement actives des immunoglobulines, celles-ci étant des molécules qui contiennent les sites de fixation spécifiques d'un antigène donné.

Le terme anticorps recouvre l'anticorps entier ainsi que des variants de l'anticorps, incluant des dérivés tels que les anticorps humanisés. Dans les anticorps naturels, deux chaînes lourdes sont liées l'une à l'autre par des ponts disulfure et chaque chaîne lourde est liée à une chaîne légère également par un pont disulfure. Il existe deux sortes de chaînes légères, les chaînes lambda (λ) et kappa (κ). Il existe cinq classes principales de chaînes lourdes qui déterminent l'activité fonctionnelle de l'anticorps : IgM, IgD, IgG, IgA et IgE. Chaque chaîne contient des domaines distincts. La chaîne légère contient deux domaines, un domaine variable (VL) et un domaine constant (CL). La chaîne lourde contient quatre ou cinq domaines selon les classes d'anticorps : un domaine variable (VH) et trois domaines à quatre domaines constants (CH1, CH2, CH3 et éventuellement CH4, regroupés sous la dénomination de CH). Les régions variables de chacune des chaînes légères (VL) et lourdes (VH) déterminent la spécificité pour l'antigène et le site de fixation sur cet antigène.

Les domaines constants des chaînes légères (CL) et lourdes (CH) confèrent à l'anticorps des propriétés biologiques importantes comme l'association des chaînes anticorps entre elles, la mobilité à travers le placenta, la fixation du complément et/ou la fixation aux récepteurs Fc (FcR). Le fragment Fv correspond à la partie *N*-terminale du fragment Fab, décrit ci-après, de l'immunoglobuline, et comprend les portions variables d'une chaîne légère et d'une chaîne lourde (VL et VH). La spécificité de l'anticorps réside dans la complémentarité structurelle entre le site de reconnaissance de l'anticorps et le déterminant antigénique. Le site de reconnaissance de l'anticorps est constitué essentiellement de résidus provenant de régions hypervariables ou déterminant la complémentarité (CDRs). Occasionnellement, les résidus provenant de régions non hypervariables ou des régions charpentes ou d'armature ("framework" ou FR) influencent la structure générale du domaine et donc les sites de reconnaissance. Le terme "régions de complémentarité" (CDR) se rapporte à des séquences d'acides aminés qui, ensemble, définissent l'affinité de la fixation et la spécificité de la région Fv naturelle du site de fixation d'une immunoglobuline native.

Chacune des chaînes légères et chacune des chaînes lourdes d'une immunoglobuline possède trois régions CDRs désignées par L-CDR1, L-CDR2, L-CDR3 et H-CDR1, H-CDR2, H-CDR3, respectivement. Le site de fixation de l'antigène inclut donc ces six CDRs, comprenant les CDRs de chacune des chaînes lourde et légère d'une région variable.

Les régions charpentes (FRs) correspondent aux séquences d'acides aminés situées entre les régions CDRs, c'est-à-dire des portions des régions variables des chaînes légères et lourdes des immunoglobulines qui sont relativement conservées entre différentes immunoglobulines d'une même espèce (Kabat *et al.* (1991) National Institutes of Health, Bethesda, Md). Le terme "région charpente humaine" est utilisé pour désigner une région charpente qui est essentiellement identique (environ 85%, préférablement 90%, 95% ou 100%) à la région charpente d'un anticorps humain naturel.

Comme il est bien connu de l'homme du métier, quatre sous-classes peuvent être distinguées parmi les anticorps de type IgG : IgG₁, IgG₂, IgG₃ et IgG₄. Ces sous-classes se distinguent par les régions constantes de leurs chaînes γ (γ₁ à γ₄). Alors qu'il n'y a pas moins de 95% d'identité de séquence entre les domaines, les régions charnières sont notablement différentes, ce qui détermine des comportements physicochimiques et des propriétés effectrices diverses.

Le terme "anticorps monoclonal" ou "mAb" (pour « monoclonal Antibody ») désigne un anticorps de composition en acides aminés unique, qui est dirigé contre un antigène spécifique et qui peut être produit par un seul clone de cellules B, ou hybridome. Les anticorps monoclonaux peuvent également être recombinants, c'est-à-dire être produits par des techniques d'ingénierie des protéines.

Le terme "anticorps anti-idiotypique" désigne un anticorps dirigé contre les déterminants antigéniques présents sur les domaines variables d'une autre immunoglobuline, soit au niveau des CDR soit en dehors.

Le terme "anticorps chimérique" signifie un anticorps recombinant qui comprend un domaine VH et un domaine VL d'un anticorps provenant d'un animal non humain, en association avec un domaine CH et un domaine CL d'un autre anticorps, en particulier d'un anticorps humain. L'animal non humain peut être une souris, un rat, un lapin, un hamster, etc. Le terme "anticorps chimérique" peut également être employé pour décrire un anticorps multispécifique, c'est-à-dire spécifique d'au moins deux antigènes différents.

Le terme d'"anticorps humanisé" signifie un anticorps humain (anticorps accepteur) dans lequel les régions de complémentarités (CDR) sont remplacées par des régions CDRs, constituant le site de liaison à l'antigène, et ayant pour origine un anticorps donneur spécifique de l'antigène. Eventuellement, l'anticorps humain accepteur reçoit, en plus des régions CDRs, des acides aminés de la région charpente de l'anticorps donneur.

### IgG₁ et ligands du récepteur CD32

L'invention concerne une immunoglobuline de type IgG₁ pour une utilisation pour le traitement d'une hypersensibilité de type 1, l'immunoglobuline étant administrée par voie mucosale, en particulier par voie sublinguale. L'immunoglobuline de type IgG₁ est un anticorps anti-IgE ayant la capacité de lier les IgE à la surface des mastocytes ou basophiles sans dissocier lesdites IgE de leurs récepteurs et de ponter les IgE fixées aux récepteurs à la surface des mastocytes ou basophiles.

Les immunoglobulines de type IgG₁ selon l'invention peuvent en particulier se trouver au sein, ou se trouver sous la forme, d'une composition d'immunoglobulines intraveineuses, aussi appelées IgIV ou IVIG.

Comme il est bien connu de l'homme du métier, les immunoglobulines intraveineuses sont des préparations thérapeutiques d'IgG humaines normales obtenues à partir d'un mélange de plasmas provenant de plus de 1000 individus sains. Il s'agit généralement quasi-exclusivement d'IgG intactes d'une demi-vie de 3 à 4 semaines et de répartitions en sous-classes semblables à celles observées dans le sérum humain normal. Les IVIG contiennent typiquement moins de 5% d'IgG agrégées, de 0 à 7% de fragments F(ab')₂ d'IgG, et, selon les préparations commerciales, de 0,06 à 40 mg d'IgA par gramme de protéines. Les IgG comprises dans les IVIG ont un large spectre de réactivités et sont ainsi dirigées contre des antigènes exogènes, des auto-antigènes et des anticorps.

Les inventeurs ayant, par ailleurs, mis en évidence une forte densité de récepteurs CD32 au niveau du chorion papillaire, qui correspond à la zone au niveau de laquelle les immunoglobulines et les protéines, en général, diffusent lorsqu'elles sont administrées par voie sublinguale, ils en ont conclu que n'importe quel ligand de CD32 était utilisable par voie sublinguale pour induire traiter des maladies et manifestations inflammatoires. Plus généralement, n'importe quel ligand de CD32 est utilisable par voie mucosale pour traiter des maladies et manifestations inflammatoires, dans la mesure où cette muqueuse présente une forte densité de récepteurs CD32.

La demande décrit également une IgG₁, ligand du récepteur CD32, de préférence un ligand du récepteur CD32B, pour une utilisation dans le traitement d'une maladie ou manifestation inflammatoire, de l'allergie ou d'une maladie auto-immune, le ligand étant administré par voie mucosale, en particulier par voie sublinguale.

Les termes "récepteur CD32" et "récepteur FcγRII" sont utilisés indifféremment et font référence à une protéine récepteur de surface présente sur la plupart des cellules immunitaires et qui se lie au fragment Fc des immunoglobulines. Comme il est bien connu de l'homme du métier, les récepteurs CD32 comprennent un groupe de récepteurs incluant les isoformes CD32A, CD32B et CD32C, toutes présentant deux domaines extracellulaires. CD32A est exprimé sur les monocytes, les macrophages, les neutrophiles et les plaquettes. CD32C est lui exprimé sur les cellules NK. CD32B enfin, qui inclut deux isoformes CD32B1 et CD32B2, est exprimé sur les lymphocytes B, les basophiles, les mastocytes, les monocytes, les macrophages et les cellules dendritiques.

CD32A initie l'endocytose, la phagocytose, la cytotoxicité cellulaire dépendante des anticorps et la libération des médiateurs de l'inflammation. CD32B transduit des signaux inhibiteurs qui régulent à la baisse les fonctions immunitaires déclenchées par les récepteurs activateurs. En particulier CD32B inhibe l'activation des mastocytes, des basophiles, des lymphocytes B et des lymphocytes T. Il est composé de 2 domaines extracellulaires de type Ig, qui lient la région Fc des IgG, un domaine transmembranaire et une queue intracytoplasmique avec un motif inhibiteur basé sur la phosphotyrosine du récepteur immunitaire (ITIM). L'activation de CD32B mène au recrutement de phosphatases au niveau du motif ITIM, qui inhibent l'activation du signal venant d'autres récepteurs activateurs.

De préférence, le récepteur CD32 correspond à l'isoforme CD32B.

Par "ligand du récepteur CD32", on entend ici des molécules se liant de manière spécifique au récepteur CD32 et induisant de préférence le signal médié par CD32.

Des techniques permettant d'identifier des ligands du récepteur CD32, en particulier des ligands du récepteur CD32B, sont bien connues de l'homme du métier. Elles incluent en particulier les techniques comprenant :
a) la mise en contact du ligand candidat avec (i) un récepteur CD32, en particulier un récepteur CD32B, recombinant ou un peptide dérivé du récepteur CD32, en particulier du récepteur CD32B; ou avec (ii) une cellule indicatrice provenant de lignées cellulaires qui expriment CD32, en particulier CD32B, à leur surface; et
b) la détermination de l'induction du signal médié par CD32, en particulier par CD32B.

Les cellules indicatrices peuvent être par exemple des cellules IIA1.6 CD32 positives (Van den Herik-Oudijk et al. (1995) Blood. 85:2202-11).

La détermination de l'induction du signal médié par CD32, en particulier médié par CD32B, peut par exemple consister à déterminer le profil de phosphorylation du motif ITIM du récepteur CD32, en particulier du récepteur CD32B, après mise en contact avec le ligand candidat. Elle peut également comprendre des tests d'inhibition de la mobilisation du calcium et/ou des tests d'inhibition de la sécrétion de cytokines, telles que IL-2.

Ainsi, des ligands candidats induisant une déphosphorylation du motif ITIM du récepteur CD32, en particulier du récepteur CD32B, et/ou inhibant la mobilisation du calcium et/ou inhibant la sécrétion d'IL-2 peuvent être considérés comme des ligands du récepteur CD32, en particulier des ligands du récepteur CD32B.

Des exemples de techniques d'identification de ligands du récepteur CD32 sont également par exemple décrits dans la demande américaine US2007/0135621.

Le ligand au récepteur CD32 selon l'invention est une IgG₁ anti-IgE ayant la capacité de lier les IgE à la surface des mastocytes ou basophiles sans dissocier lesdites IgE de leurs récepteurs et de ponter les IgE fixées aux récepteurs à la surface des mastocytes ou basophiles.

Une immunoglobuline de type IgG₁ peut en particulier être un anticorps monoclonal, un anticorps chimérique ou un anticorps humanisé, tels que définis ci-dessus.

Dans un mode de réalisation préféré, lorsque l'immunoglobuline selon l'invention est un anticorps chimérique tel que défini ci-dessus, les domaines CH et CL associés aux domaines VH et VL proviennent d'une immunoglobuline humaine de type IgG₁.

Dans un autre mode de réalisation particulièrement préféré, l'immunoglobuline se liant au récepteur CD32 se lie au récepteur via sa région Fc.

Les inventeurs ont montré que, de manière surprenante, des immunoglobulines de n'importe quel idiotype, et pas seulement des anticorps anti-IgE, étaient capables d'induire une amélioration des symptômes d'asthme allergique lorsqu'elles étaient administrées par voie sublinguale.

Par "allergène", on entend ici toute substance déclenchant une allergie. Des exemples d'allergènes peuvent être, à titre non limitatif, des allergènes de pollen (d'arbres, de graminées, etc.), des allergènes d'acarien (de la poussière domestique ou de stockage), des allergènes d'insecte (d'hyménoptères, de blattes, etc.), des allergènes d'animaux (de chien, de chat, de cheval, de rat, de souris, etc.), des allergènes de moisissure et des allergènes alimentaires. L'allergène peut par exemple être un des allergènes listés dans la section « Applications thérapeutiques ».

Par « antigène », on entend toute substance capable de déclencher une réponse immunitaire visant à l'éliminer de l'organisme. Il s'agit généralement d'une substance étrangère à l'organisme. Dans le cas des maladies auto-immunes, l'antigène peut être un antigène du « soi » reconnu à tort comme étranger par l'organisme (auto-antigène).

Par "cytokine", on entend ici toute protéine, autre qu'un anticorps, sécrétée par une cellule au contact d'un antigène spécifique et jouant un rôle de médiateur cellulaire dans la génération d'une réponse immunitaire. Des exemples de cytokines peuvent être, à titre non limitatif, des interférons, tels que IFN-α, IFN-β, IFN-ω et IFN-γ; des interleukines, telles que IL-1, IL-2, IL-3, IL-4, IL-5, IL-6, IL-7, IL-8, IL-9, IL-10, IL-11, IL-12, IL-13, IL-17, IL-18 et IL-23; des chimiokines telles que les chimiokines de la famille CXC, les chimiokines de la famille CC, les chimiokines de la famille CX3C et les chimiokines de la famille C; le TNF, les CSF et les TGF.

L'immunoglobuline selon l'invention est un anticorps anti-IgE de type IgG1 ayant la capacité de lier les IgE à la surface des mastocytes ou basophiles sans dissocier lesdites IgE de leurs récepteurs et de ponter les IgE fixées aux récepteurs à la surface des mastocytes ou basophiles.

Les inventeurs ont par ailleurs montré de manière surprenante qu'il était possible d'induire une amélioration des symptômes allergiques en utilisant des immunoglobulines, en particulier des IgG₁, qu'elles soient sous forme sialylée ou non sialylée.

Ainsi, dans un mode de réalisation particulièrement préféré de l'invention, les immunoglobulines selon l'invention ne sont pas sous forme sialylée.

Par "sialylation", on entend ici l'addition d'un résidu d'acide sialique sur une protéine, qui peut être en particulier une glycoprotéine.

Dans le contexte de l'invention, le terme "acide sialique" inclut une famille de sucres contenant 9 atomes de carbone ou plus, comprenant un groupement carboxyle. Une structure générique englobant toutes les formes naturelles d'acide sialique est montrée dans la formule (I) ci-dessous: 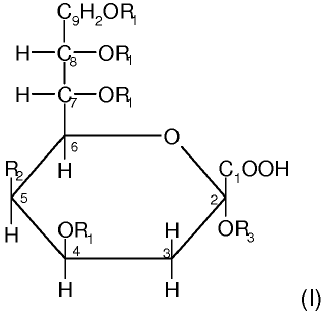 dans laquelle
les groupements R₁ à des positions variées sur une seule molécule peuvent être identiques ou différents les uns des autres. R₁ peut être un hydrogène ou un groupement acétyle, lactyle, méthyle, sulfate, phosphate, anhydro, acide sialique, fucose, glucose ou galactose;
R₂ peut être un groupement N-acétyle, N-glycolyle, amino, hydroxyle, N-glycolyl-O-acétyle ou N-glycolyl-O-méthyle;
R₃ représente le résidu de sucre précédent dans un oligosaccharide auquel l'acide sialique est attaché dans le contexte d'une glycoprotéine. R₃ peut être un galactose (connecté en position 3, 4, 5 ou 6), une N-acétyl-galactosamine (connecté en position 6), une N-acétyl-glucosamine (connecté en position 4 ou 6), un acide sialique (connecté en position 8 ou 9) ou un acide 5-N-glycolyl-neuraminique.

On trouve plus de 40 formes d'acide sialique dans la nature parmi lesquelles l'acide N-acétylneuraminique, l'acide N-glycolylneuraminique et leurs dérivés O-acétylés, en particulier l'acide N-acétyl-9-O-acétylneuraminique. La forme d'acide sialique la plus commune est l'acide N-acétylneuraminique, dans lequel R₁ est un hydrogène à toutes les positions et R₂ est un groupement N-acétyle.

Dans le contexte de l'invention, on entend par "immunoglobuline sous forme sialylée", une immunoglobuline qui contient au moins un acide sialique tel que défini ci-dessus.

Dans le contexte de l'invention, on entend par "immunoglobuline qui n'est pas sous forme sialylée" ou "immunoglobuline sous forme non-sialylée", une immunoglobuline qui ne contient pas d'acide sialylique tel que défini ci-dessus.

### Anticorps anti-IgE ayant la capacité de lier les IgE fixées sur leurs récepteurs à la surface d'un mastocyte et/ou basophile

L'invention concerne par ailleurs un anticorps anti-IgE de type IgG1 ayant la capacité de lier les IgE fixées sur leurs récepteurs à la surface d'un mastocyte ou basophile sans dissocier lesdites IgE de leurs récepteurs et la capacité de ponter les IgE fixées aux récepteurs à la surface des mastocytes ou basophiles, destiné à être utilisé dans le traitement d'une allergie immédiate ou hypersensibilité de type 1.

Par "récepteur aux IgE" on entend le récepteur RFcεl, présent à la surface d'un mastocyte et/ou basophile.

Un anticorps anti-IgE selon l'invention peut en particulier être un anticorps monoclonal, un anticorps chimérique ou un anticorps humanisé, tels que définis ci-dessus.

Plus particulièrement, l'anticorps anti-IgE selon l'invention peut être un anticorps anti-idiotypique ou non. Ainsi, l'anticorps anti-IgE selon l'invention peut se lier à n'importe quelle IgE, quelle que soit la spécificité de cette IgE, ou au contraire l'anticorps anti-IgE ne peut se lier qu'à une IgE spécifiquement dirigée contre un antigène ou un allergène particulier.

Dans un mode de réalisation préféré, lorsque l'anticorps anti-IgE selon l'invention est un anticorps chimérique tel que défini ci-dessus, les domaines VH et VL associés aux domaines CH et CL proviennent d'un anticorps dirigé contre les IgE humaines.

Dans un autre mode de réalisation préféré, lorsque l'anticorps anti-IgE selon l'invention est un anticorps humanisé tel que défini ci-dessus, l'anticorps donneur est un anticorps de souris, de rat, de lapin, d'hamster, etc. et est spécifique des IgE humaines. Par exemple, des CDRs d'un anticorps de souris spécifique des IgE humaines peuvent être greffés dans la région charpente d'un anticorps humain pour préparer un "anticorps humanisé" spécifique des IgE humaines.

Préférentiellement, l'anticorps anti-IgE selon l'invention est un anticorps humain ou humanisé.

Les IgE sont capables de se lier par leurs parties constantes Fc aux récepteurs Recεl présents à la surface des mastocytes et basophiles. L'anticorps anti-IgE selon l'invention a la capacité de se lier à des IgE fixées aux récepteurs RFcεl d'un mastocyte ou basophile sans dissocier lesdites IgE de leurs récepteurs.

Des recherches antérieures ont porté sur les régions impliquées dans la liaison des IgE avec les récepteurs RFcεl. Ces régions ont été identifiées comme étant le domaine Cε3 et partiellement le domaine Cε4 (Schwarzbaum et al. (1989) Eur. J. Immunol. 19 : 1015-1023), ou le domaine Cε2, Cε3 et la région entre les domaines Cε2 et Cε3 (Takemoto et al. (1994) Microbiol. Immunol. 38 : 63-71).

Il est donc préférable, selon l'invention, que l'anticorps anti-IgE reconnaisse une partie de l'IgE non impliquée dans la fixation de l'IgE à son récepteur sur les mastocytes ou basophiles. De façon préférentielle, l'anticorps anti-IgE selon l'invention peut reconnaître les parties des domaines Cε2, Cε3 Cε4 non impliquées dans la liaison au récepteur RFcεl, le domaine Cε1, les domaines variables de la chaîne lourde (VH) ou de la chaîne légère (VL) ainsi que le domaine constant de la chaîne légère des IgE (CL).

D'une manière générale, l'homme du métier peut vérifier la capacité d'un anticorps donné à se fixer aux IgE liées au récepteur RFcεl par des méthodes usuelles dans le domaine technique. Par exemple l'homme du métier peut vérifier que l'ajout d'anticorps anti-IgE n'empêche pas la fixation de l'IgE sur les récepteurs RFcεl à la surface de basophiles ou mastocytes. Selon sa spécificité, l'anticorps anti-IgE selon l'invention peut éventuellement empêcher la reconnaissance de l'allergène par l'IgE, par exemple en créant un encombrement stérique dans le site de reconnaissance de l'antigène.

L'anticorps anti-IgE a la capacité de ponter des IgE fixées sur leurs récepteurs à la surface d'un mastocyte ou d'un basophile.

Le terme "pontage des IgE" se réfère à l'établissement de liaisons croisées entre deux IgE liées chacune à un récepteur RFcεl à la surface d'un mastocyte ou d'un basophile. Ces liaisons croisées sont provoquées par la reconnaissance d'au moins deux IgE, liés à des récepteurs RFcεl sur la même cellule, par une même molécule pontante (allergène, anticorps anti-IgE) et induisent l'activation des mastocytes ou basophiles.

Lorsqu'une quantité suffisante d'IgE de surface est pontée, c'est-à-dire lorsqu'une quantité suffisante, dite « quantité optimale », de molécules pontantes est présente, les liaisons croisées peuvent induire la dégranulation des mastocytes et basophiles et entraîner la libération de médiateurs pro-inflammatoires.

Cette capacité de pontage des IgE peut être évaluée par l'homme du métier, par exemple par un test cellulaire permettant de détecter la dégranulation ou l'activation des mastocytes ou des basophiles en présence d'une quantité optimale d'anticorps anti-IgE. La dégranulation peut par exemple être estimée par la mesure de la quantité d'histamine ou de β-hexosaminidase libérée lors de cette dégranulation ou par la mesure, en cytométrie de flux par exemple, de la présence de CD63 ou/et CD107a à la surface des mastocytes ou des basophiles.

Alternativement, on peut également mesurer l'activation des cellules suite au pontage en présence d'anticorps anti-IgE en cytométrie de flux, en mesurant la présence de CD203c à la surface des mastocytes ou des basophiles. L'anticorps anti-IgE peut dans ce cas être testé à une concentration « sub-optimale » qui se réfère ici à une concentration d'anticorps anti-IgE insuffisante pour induire la dégranulation des mastocytes ou basophiles.

Les anticorps anti-IgE selon l'invention sont spécifiques d'une IgE et capables de se lier à des IgE fixées aux récepteurs RFcεl d'un mastocyte ou d'un basophile sans dissocier lesdites IgE de leurs récepteurs, et préférentiellement capables d'induire le pontage des IgE fixées sur les récepteurs RFcεl des mastocytes et basophiles.

L'anticorps anti-IgE selon l'invention peut en particulier être un anticorps ayant la capacité de se lier au récepteur CD32, en particulier au récepteur CD32B, tel que défini ci-dessus. L'anticorps anti-IgE selon l'invention est un anticorps de type IgG₁.

A titre d'exemple d'anticorps spécifiques d'IgE murines, on peut citer l'IgG1κ issue du clone R35-72 (BD Biosciences Pharmingen, San Diego, CA, USA), l'IgG1κ issue du clone 23G3 (Southern Biotechnology Associates, Birmingham, AL. Posner et al. (2004) Biochemestry 43 : 11352-11360, Keegan et al. (1991) Mol. Immunol. 28 : 1149-1154), les IgG2a issues des clones E11AC2IIC3, E11BA1ID1, E11BA3ID4, E9AD2IIA5, E11BB5IA6, E5BB3IIA4 et l'IgG1 issue du clone E5AA1IA6 (Hook et al (1991) Mol. Immunol. 6 : 631-639), l'IgG2a issue du clone C12B9 (Keegan et al. (1991) Mol. Immunol. 28 : 1149-1154), l'IgG2a issue du clone LO-ME-2 (Kang et al. (2007) Immune Network 7 : 141-148) ou un fragment ou une forme humanisée de ceux-ci.

A titre d'exemple d'anticorps pontant spécifique d'IgE humaines, on peut citer l'IgG1 issue du clone E124.2.8 (Beckman Coulter).

Les anticorps peuvent, dans un mode de réalisation préféré, être des anticorps chimériques ou humanisés dérivés de l'anticorps cité ci-dessus.

Les anticorps anti-IgE selon l'invention sont administrés par voie mucosale. De manière davantage préférée, les anticorps anti-IgE selon l'invention sont administrés par voie sublinguale.

### Méthodes de production d'anticorps

Les anticorps selon l'invention peuvent être produits par toute technique connue, telle que, par exemple, des techniques chimiques, biologiques, génétiques, enzymatiques ou des combinaisons de celles-ci.

Les anticorps chimériques ou humanisés selon l'invention peuvent être obtenus par tous moyens connus de l'homme du métier, en particulier par ingénierie génétique des anticorps.

La construction d'un anticorps chimérique consiste à isoler l'ADN codant pour la région VH et la région VL d'un anticorps monoclonal donneur et à le lier à l'ADN codant les régions constantes CH et CL d'une immunoglobuline humaine.

Un anticorps humanisé s'obtient en remplaçant les régions hypervariables d'un anticorps monoclonal receveur par les régions hypervariables d'un anticorps donneur ("CDR grafting") et peut éventuellement nécessiter les étapes suivantes :
i) Conception de l'anticorps humanisé :
   - détermination des régions CDRs de l'anticorps donneur devant être transférées, et éventuellement, identification des résidus des régions charpentes de l'anticorps donneur devant également être transférés en raison de leurs rôles dans le maintien de la structure des CDRs, ou de leurs contributions au site de liaison à l'antigène. Ces régions ou résidus peuvent être identifiés lors de la construction d'un modèle 3D des régions variables de l'anticorps et par utilisation de logiciels informatiques tel que RASMOL,
   - identification dans des bases de données de l'anticorps humain le plus approprié à l'humanisation et choix de l'isotype humain. Par exemple un anticorps approprié à l'humanisation peut être un anticorps ayant des régions charpentes proches de celles de l'anticorps donneur.
ii) synthèse des régions variables ainsi conçues par exemple par amplification PCR de séquences chevauchantes et obtention des anticorps humanisés.

L'homme du métier qui connaît la composition en acides aminés de la séquence désirée est capable de produire des anticorps par des techniques standardisées de production de polypeptides. Par exemple, ils peuvent être synthétisés en phase solide, préférentiellement en utilisant un appareil de synthèse de peptides commercialisé, tel celui produit par Applied Biosystems, Californie (USA), et en suivant les instructions du producteur.

Alternativement, les anticorps selon l'invention peuvent être produits par des techniques d'ADN recombinant dans un système d'expression adapté. Le terme "système d'expression" signifie un hôte cellulaire et un vecteur compatible dans des conditions appropriées, c'est-à-dire des conditions permettant l'expression de la protéine codée par l'ADN étranger porté par le vecteur et introduit dans la cellule hôte. Typiquement, la séquence d'acides nucléiques codant un anticorps peut être insérée dans un vecteur d'expression approprié qui sera alors introduit dans un hôte procaryote ou eucaryote adéquat qui produira l'anticorps désiré.

Les termes "vecteur", "vecteur de clonage" et "vecteur d'expression" se rapportent à des véhicules grâce auxquels les séquences d'ADN ou d'ARN codant l'anticorps peuvent être introduites dans une cellule hôte de façon à la transformer et à permettre l'expression (c'est-à-dire la transcription et la traduction) de la séquence introduite. Un vecteur d'expression est typiquement un plasmide, un cosmide, un épisome, un chromosome artificiel, un phage ou un vecteur viral.

A titre de vecteurs viraux on peut citer les adénovirus, les rétrovirus, le virus de l'herpès et les vecteurs dérivés du virus adéno-associé (AAV). De tels virus recombinants peuvent être produits par des techniques bien connues, comme la transfection de lignées cellulaires permettant leur encapsidation ou par transfection transitoire avec des plasmides ou des virus de complémentation exprimant les fonctions manquantes nécessaires. On peut citer par exemple, les lignées de cellules permettant l'encapsidation PA317, PsiCRIP, GPenv+, 293, etc. Les protocoles détaillés permettant de produire de tels virus recombinants défectueux pour la réplication sont disponibles dans les demandes de brevet WO 95/14785, WO 96/22378, US 5,882,877, US 6,013,516, US 4,861,719, US 5,278,056 et WO 94/19478.

Les cellules hôtes sont transfectées, infectées ou transformées par un acide nucléique ou un vecteur approprié tel que décrit ci-dessus. Le terme de transformation se rapporte à l'introduction d'un gène étranger (extrinsèque ou extracellulaire), d'une séquence d'ADN ou d'ARN dans une cellule hôte de telle manière que cette cellule hôte exprime le gène introduit, la séquence d'ADN ou d'ARN pour produire la substance désirée, typiquement la protéine codée par le gène ou la séquence introduite.

Des systèmes d'expression usuels incluent des cellules hôtes et des vecteurs plasmidiques d'*E. coli,* des cellules hôtes d'insectes et des vecteurs de type Baculovirus et des cellules et des vecteurs de mammifères.

Une méthode de production à partir d'une cellule hôte exprimant un anticorps selon l'invention peut comprendre les étapes consistant à : (i) introduire in vitro ou *ex vivo* un acide nucléique recombinant ou un vecteur tel que décrit ci-dessus dans la cellule hôte compétente, (ii) cultiver in vitro la cellule hôte recombinante ainsi obtenue, (iii) éventuellement sélectionner les cellules qui expriment et/ou secrètent ledit anticorps ou polypeptide.

De plus, une méthode de production de l'anticorps selon l'invention peut comprendre les étapes consistant à: (i) cultiver la cellule transformée décrite ci-dessus dans des conditions appropriées à l'expression de l'anticorps ; et (ii) récupérer l'anticorps ainsi exprimé.

Les anticorps peuvent être séparés du milieu de culture par des méthodes conventionnelles de purification des immunoglobulines telles que, par exemple, purification sur protéine A-Sepharose, par chromatographie sur hydroxylapatite, par électrophorèse sur gel, par dialyse ou par chromatographie d'affinité.

### Compositions thérapeutiques

La présente invention concerne également des compositions pharmaceutiques pour le traitement d'une hypersensibilité de type 1 par voie mucosale, de manière davantage préférée par administration par voie sublinguale, comprenant un anticorps anti-IgE ayant la capacité de lier les IgE fixées sur leurs récepteurs à la surface d'un mastocyte ou basophile sans dissocier lesdites IgE de leurs récepteurs et la capacité de ponter les IgE fixées aux récepteurs à la surface des mastocytes ou basophiles, tel que défini ci-dessus.

Dans un mode de réalisation particulier, la composition pharmaceutique selon l'invention peut comprendre en outre un allergène tel que défini ci-dessus, ou un antigène.

Les anticorps selon l'invention peuvent être combinés à des excipients pharmaceutiquement acceptables et, éventuellement, à des matrices à libération prolongée comme, par exemple, des polymères biocompatibles pour former des compositions thérapeutiques. De tels polymères peuvent par exemple être des polysaccharides comme l'amidon, les pectines, les amylopectines, le chitosan ou des particules ou des particules polysaccharidiques (PSC) tels que décrites par Razafindratsita et al. (2007, J. Allergy Clin Immunol. 120: 278-285) et pouvant être sous formes de nano-particules ou bien de micro-particules.

Pour des thérapies combinées, les compositions pharmaceutiques peuvent comprendre à la fois les anticorps selon l'invention et un traitement de désensibilisation spécifique et/ou un traitement symptomatique de l'allergie. A titre d'exemples de traitement de désensibilisation spécifique on peut citer tout traitement comportant l'administration d'extraits allergéniques. Les traitements symptomatiques peuvent comprendre des substances anti-inflammatoires (comme des corticostéroïdes ou des antihistaminiques), des inhibiteurs de leucotriènes, des broncho-dilatateurs, le cromoglycate de sodium, la théophylline.

Le terme "pharmaceutiquement acceptable" se rapporte à des molécules et compositions qui n'induisent pas une réaction adverse allergique ou non désirée lorsqu'elles sont administrées à un mammifère, en particulier à un humain. Un véhicule ou excipient pharmaceutiquement acceptable peut être un solide ou un semi-solide, un liquide, un diluant, un matériel encapsulé ou n'importe quelle autre formulation.

La forme de la composition pharmaceutique, le mode d'administration, le dosage et la posologie peuvent bien sûr dépendre, entre autres, de la maladie à traiter, de ses symptômes, de sa sévérité, de l'âge, du poids et du sexe du patient.

La composition pharmaceutique ou thérapeutique selon l'invention est formulée de façon à pouvoir être administrée par voie mucosale, sublinguale, orale, nasale, vaginale, rectale, bronchique, auriculaire, conjonctivale,. De préférence, la composition pharmaceutique ou thérapeutique est administrée par voie sublinguale, orale, nasale, vaginale, rectale, bronchique, auriculaire, ou conjonctivale. De manière préférée, la composition pharmaceutique ou thérapeutique est administrée par voie mucosale, en particulier par voie buccale, sublinguale, nasale, orale, bronchique, rectale, vaginale ou auriculaire. De manière préférée entre toutes, la composition pharmaceutique ou thérapeutique est administrée par voie sublinguale.

Les compositions pharmaceutiques selon l'invention, peuvent éventuellement contenir des excipients pharmaceutiquement acceptables appropriés pour qu'elles puissent être injectées (en particulier ceux-ci peuvent être des solutions salines isotoniques et stériles, phosphate monosodique ou disodique, chlorure de sodium, potassium, calcium ou magnésium, etc., ou mélange de ces sels). Ces compositions peuvent également être des compositions sèches, en particulier des compositions sèches et congelées, lyophilisées ou réfrigérées, qui après addition, selon les cas, d'eau stérile ou d'eau physiologique, constituent des solutions injectables.

Les doses utilisées peuvent être adaptées en fonction de différents paramètres comme, en particulier, le mode d'administration, le type de pathologie ou alternativement, la durée du traitement envisagée. Préférentiellement la dose est adaptée pour permettre un traitement en une seule fois par monodose.

Pour préparer les compositions pharmaceutiques, une quantité suffisante d'anticorps peut être dissoute ou dispersée dans un véhicule pharmaceutiquement acceptable ou un milieu aqueux.

Les formes pharmaceutiques appropriées à une utilisation par injection comprennent les solutions d'eau stérile, les dispersions, les formulations incluant de l'huile de sésame, ou du propylène glycol aqueux, ainsi que des poudres stériles pour la préparation extemporanée de solutions injectables stériles. Dans tous les cas, la forme utilisée doit être stérile et doit être suffisamment fluide pour pouvoir être injectée facilement au moyen d'une seringue. Elle doit être stable dans les conditions de production et de stockage et être protégée des contaminations par des microorganismes, tels que les bactéries ou les champignons.

Les solutions des composés actifs, qu'ils soient sous forme libre ou en tant que sels acceptables d'un point de vue pharmaceutique, peuvent être préparées avec de l'eau mélangée à un surfactant comme l'hydroxypropylcellulose. Les dispersions peuvent être faites dans du glycérol, dans des polyéthylènes glycols liquides, dans un mélange des deux ou dans des huiles. Ces préparations contiennent généralement un agent conservateur pour empêcher la croissance de micro-organismes dans les conditions normales de stockage et d'utilisation.

Après formulation sous forme de médicament, les solutions peuvent être administrées d'une manière compatible avec le dosage de la formulation et en une quantité thérapeutiquement active. Les médicaments peuvent être administrés comme décrit ci-dessus mais également sous forme de capsules qui les libèrent.

L'administration par voie sublinguale est particulièrement préférée. Lors d'une administration sublinguale, la composition pharmaceutique atteint les cellules de la muqueuse et éventuellement les cellules de la sous-muqueuse. Ce mode d'administration a pour avantage d'être simple, rapide et d'éviter le passage par le tractus gastro-intestinal ou la composition pharmaceutique risque d'être dégradée par les enzymes digestives. Une composition pharmaceutique pour administration par voie sublinguale peut préférentiellement être formulée sous forme de gouttes (pouvant contenir du glycérol) ou de comprimés.

De préférence, lorsque les anticorps et/ou les compositions pharmaceutiques selon l'invention sont administrés par voie mucosale, en particulier par voie sublinguale, ils ne sont pas directement accessibles aux récepteurs DC-SIGN. Par "DC-SIGN" (en anglais "*Dendritic Cell-Specific Intercellular adhesion molecule-3-Grabbing Non-integrin")*, ou "CD209", on entend ici une lectine de type C ayant une forte affinité pour l'acide sialique.

Les immunoglobulines selon l'invention peuvent être ajoutées à un mélange thérapeutique en une quantité d'environ 0,001 à 1000 milligrammes ou d'environ 0,01 à 500 milligrammes ou d'environ 1 à 200 milligrammes, d'environ 10 à 100 milligrammes par dose, préférentiellement de l'ordre de 100 milligrammes. Des doses multiples peuvent également être administrées.

De manière préférée, l'immunoglobuline selon l'invention est administrée à une concentration suboptimale, c'est-à-dire à une concentration insuffisante pour induire la dégranulation des mastocytes ou basophiles. La concentration suboptimale d'un anticorps selon l'invention peut être déterminée aisément par l'homme du métier. Par exemple, celui-ci peut tester l'effet de différentes concentrations d'anticorps sur des mastocytes et basophiles. La concentration suboptimale correspondra à toutes concentrations inférieures à la concentration minimale (concentration optimale) induisant la dégranulation. Préférentiellement, la concentration suboptimale est déterminée lors d'essais cliniques chez l'homme ou l'animal.

D'autres formes pharmaceutiques sont acceptables incluant par exemple les comprimés ou d'autres formes solides d'administration, capsules à relargage retardé ou toute autre forme utilisable.

Dans certains modes de réalisation de l'invention, l'utilisation de liposomes et/ou de microparticules et/ou de nanoparticules est envisageable pour introduire des anticorps dans l'hôte. L'utilisation et la formation de liposomes et/ou de microparticules et/ou de nanoparticules sont connues de l'homme du métier.

### Applications thérapeutiques

La demande décrit des immunoglobulines utilisées pour traiter des maladies et manifestations inflammatoires.

Par "maladie inflammatoire", on entend ici une maladie associée à une inflammation. Des exemples de maladies inflammatoires sont bien connus de l'homme du métier et incluent en particulier l'asthme, les hypersensibilités, les allergies.

Les « manifestations inflammatoires » désignent réactions inflammatoires qui se produisent au cours de maladies qui, elles, ne sont pas à proprement parler inflammatoires comme par exemple les parasitoses ou les mastocytoses.

Les immunoglobulines ayant la capacité de lier les IgE à la surface des mastocytes ou basophiles sans dissocier lesdites IgE de leurs récepteurs et de ponter les IgE fixées aux récepteurs à la surface des mastocytes ou basophiles pour l'utilisation selon l'invention, et les composés ou les médicaments les comprenant, sont utilisés pour traiter les allergies immédiates.

Le terme "allergie immédiate" ou "hypersensibilité de type I" tel qu'utilisé ici signifie une réponse humorale en réponse à un allergène qui se distingue d'une réponse humorale normale par le fait que les plasmocytes sécrètent des IgE.

Parmi les manifestations cliniques dues aux allergies immédiates pouvant être traitées par l'anticorps selon l'invention on peut citer, à titre d'exemples, l'anaphylaxie systémique, l'anaphylaxie localisée (atopie), la rhinite allergique, l'asthme, les allergies alimentaires, la dermatite atopique, la conjonctivite, l'eczéma, la mastocytose induite par un choc anaphylactique, les manifestations allergiques causées par les IgE sécrétées en réponse à une infection par un parasite. De manière particulièrement préférée, dans le cadre de l'invention, les allergies immédiates se manifestent par de l'asthme.

Dans le cadre de l'invention l'allergie peut être causée par exposition d'un individu à n'importe quel allergène.

Des exemples d'allergènes peuvent être, à titre non limitatif, des allergènes de pollen (d'arbres, de graminées, etc.), des allergènes d'acarien (de la poussière domestique ou de stockage), des allergènes d'insecte (hyménoptères, de blattes, etc.), des allergènes d'animaux (de chien, de chat, de cheval, de rat, de souris, etc.), des allergènes de moisissure et des allergènes alimentaires.

Par exemple, l'allergène peut être sélectionné dans le groupe consistant en les protéines allergènes du genre *Dermatophagoides,* les protéines allergènes du genre *Felis,* les protéines allergènes du genre *Ambrosia,* les protéines allergènes du genre *Lolium,* les protéines allergènes du genre *Cryptomeria,* les protéines allergènes du genre *Alternaria,* les protéines allergènes du genre *Alder,* les protéines allergènes du genre *Betula,* les protéines allergènes du genre *Blomia,* les protéines allergènes du genre *Quercus,* les protéines allergènes du genre *Olea,* les protéines allergènes du genre *Artemisia,* les protéines allergènes du genre *Plantago,* les protéines allergènes du genre *Parietaria,* les protéines allergènes du genre *Canis,* les protéines allergènes du genre *Blattella,* les protéines allergènes du genre *Apis,* les protéines allergènes du genre *Cupressus,* les protéines allergènes du genre *Thuya,* les protéines allergènes du genre *Chamaecyparis,* les protéines allergènes du genre *Periplaneta,* les protéines allergènes du genre *Agropyron,* les protéines allergènes du genre *Secale,* les protéines allergènes du genre *Triticum,* les protéines allergènes du genre *Cynorhodon,* les protéines allergènes du genre *Juniperus,* les protéines allergènes du genre *Dactylis,* les protéines allergènes du genre *Festuca,* les protéines allergènes du genre *Poa,* les protéines allergènes du genre *Avena,* les protéines allergènes du genre *Holcus,* les protéines allergènes du genre *Anthoxanthum,* les protéines allergènes du genre *Arrhenatherum,* les protéines allergènes du genre *Agrostis,* les protéines allergènes du genre *Phleum,* les protéines allergènes du genre *Phalaris,* les protéines allergènes du genre *Paspalum,* les protéines allergènes du genre *Sorghum.* Des exemples de protéines allergènes connues dérivant de protéines des genres énumérés ci-dessus incluent : *Cynorhodon* Cyn d 1 ; *Dermatophagoides (pteronyssinus* ou *farinae)* Der p 1 ; Der p 2 ; Der p 3 ; Der p 5 ; Der p 7 ; Der f 1 ; Der f 2 ; Der f 3 ; Der f 5 ; Der f 7 ; *Felis (domesticus)* Fel d 1 ; *Ambrosia* (*artemisiifolia*) Amb a 1 ; Amb a 2 ; Amb a 3 ; Amb a 4 ; *Lolium (perenne)* Lol p 1 ; Lol p 2 ; Lol p 3 ; Lol p 4 ; Lol p 5 ; Lol p 9 ; *Cryptomeria* (*japonica*) Cry j 1 ; Cry j 2 ; *Juniperus* (*sabinoides* ou *virginiana*) Jun s 1 ; Jun v 1 ; *Juniperus* (*ashei*) Jun a 1 ; Jun a 2 ; *Dactylis* (*glomerata*) Dac g 1 ; Dac g 5 ; *Poa* (*pratensis*) Poa p 1 ; Poa p 5 ; *Phleum* (*pratense*) Phl p 1 ; Phl p 5 ; *Anthoxanthum (odoratum)* Ant o 1 ; Ant o 5 ; *Betula (verrucosa)* Bet v 1 ; Bet v 2 ; Bet v 4 and *Sorghum* (*halepensis*) Sor h 1.

Les allergènes alimentaires peuvent provenir du lait, des oeufs, des légumes (dont cacahuète et soja), des noix et noisettes, du blé, de crustacés, de poissons et de mollusques et des produits qui en sont dérivés. En particulier, les allergènes alimentaires peuvent être l'ovalbumine ou le gluten.

Dans le contexte de l'invention, le terme "traiter" ou "traitement" signifie supprimer, alléger ou empêcher la progression d'un désordre ou prévenir l'apparition d'un tel désordre ou d'un ou plusieurs symptômes liés à ce désordre. En particulier, le traitement des allergies immédiates peut consister à réduire ou même préférentiellement à supprimer l'inflammation excessive due à la libération par les mastocytes et basophiles de molécules pro-inflammatoires par l'administration d'une quantité thérapeutiquement active d'anticorps selon l'invention.

Le terme "patient" ou "individu" selon l'invention est utilisé pour désigner un humain ou un mammifère non humain (tel que par exemple un rongeur (souris, rat), un félin, un canidé ou un primate) qui développe ou risque de développer une allergie immédiate. Préférentiellement, le sujet est un humain.

Le terme "quantité thérapeutiquement active" signifie une quantité d'anticorps suffisante pour traiter une allergie immédiate et ayant un rapport bénéfice/risque acceptable pour un traitement médicamenteux. La quantité d'anticorps et de compositions selon la présente invention ainsi que la fréquence d'administration sera déterminée par des études cliniques, par le médecin ou par le pharmacien. La dose "thérapeutiquement active" spécifique à chacun des patients pourra dépendre d'un certain nombre de facteurs comme la nature et la sévérité du désordre à traiter, l'activité de l'anticorps utilisé, la composition utilisée, l'âge, le poids, l'état de santé général, le sexe et le régime du patient, le mode d'administration, la durée du traitement (en monodose ou en plusieurs doses), les médicaments utilisés en combinaison et d'autres facteurs bien connus des spécialistes médicaux.

L'invention est également décrite dans les figures et exemples suivants, qui n'ont pas de caractère limitatif.

### FIGURES

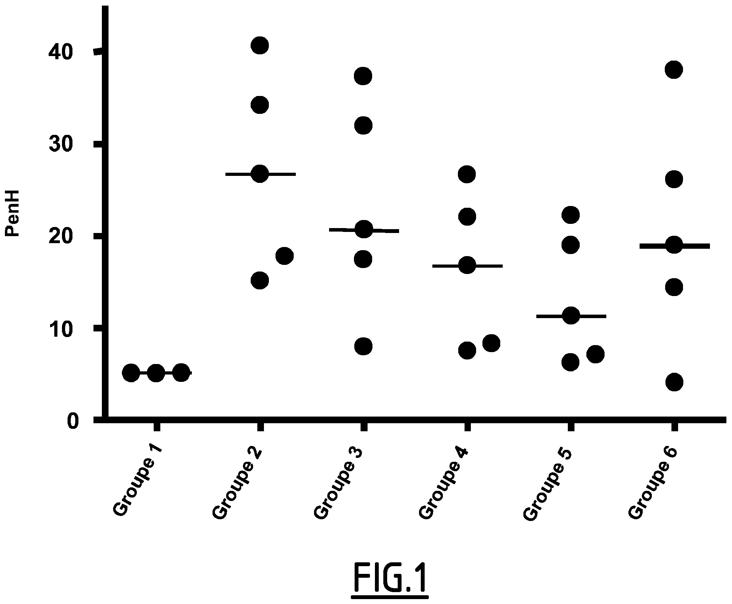

La Figure 1 représente l'effet, sur l'hyperréactivité bronchique (mesurée en valeur de PenH ou « enhanced pause »), de la stimulation à la métacholine d'un groupe de trois souris BALB/c non sensibilisées à l'ovalbumine (groupe 1) et de groupes de cinq souris après sensibilisation à l'ovalbumine et désensibilisation avec :
   - groupe 2 : du PBS ;
   - groupe 3 : de l'ovalbumine à raison de 500 µg par administration sublinguale, deux fois par semaine, pendant deux mois ;
   - groupe 4: l'isotype contrôle de rat de type IgG1κ à raison de 25 μg par administration sublinguale, deux fois par semaine, pendant deux mois ;
   - groupe 5 : de l'anticorps de rat anti-IgE de souris de clone R35-72 à raison de 25 μg par administration sublinguale deux fois par semaine pendant deux mois ;
   - groupe 6 : de l'anticorps de rat anti-IgE de souris de clone R35-72, à raison de 10 μg par administration sublinguale, deux fois par semaine, pendant deux mois.

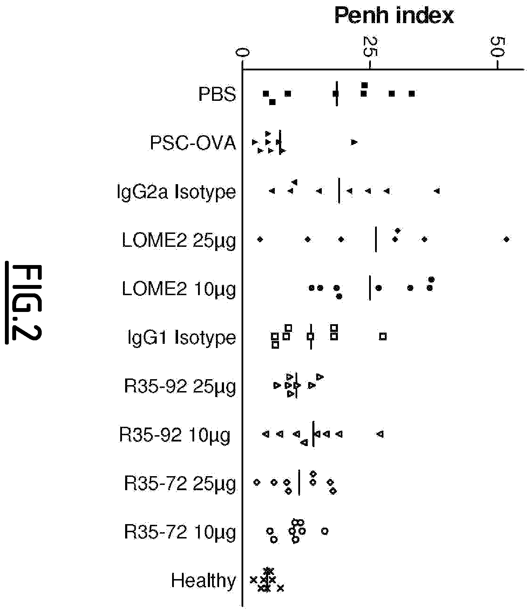

La Figure 2 montre la réactivité des voies respiratoires par mesure de valeur Penh en réponse à une administration de métacholine (100 mg/ml). Huit souris ont été analysées dans chaque groupe. Les barres horizontales représentent la réponse moyenne au sein de chaque groupe, chaque point représentant la valeur Penh obtenue pour un animal donné. Les résultats sont représentatifs de deux expériences indépendantes.

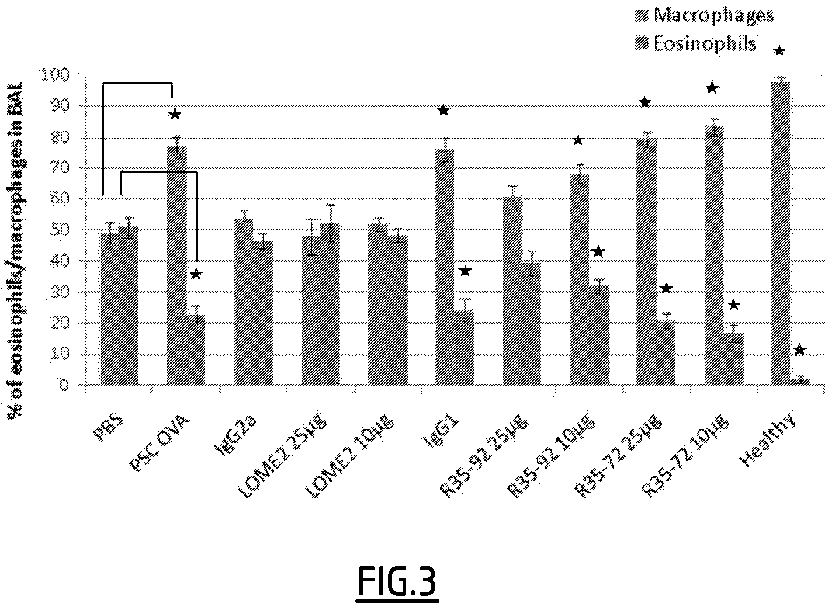

La Figure 3 montre le décompte de macrophages et éosinophiles dans des lavages broncho-alvéolaires de souris recevant divers traitements. Les résultats sont représentés en tant que moyenne ± erreur standard de la moyenne. N = 8 souris par groupe. * p< 0,05 en comparaison de souris désensibilisées avec du PBS (placebo). Les données ont été comparées en utilisant le test non paramétrique (Kruskal-Wallis).

### EXEMPLE 1

### Matériels et méthodes

### Sensibilisation et désensibilisation des souris.

Des souris BALB/c ont été sensibilisés avec de l'ovalbumine (OVA) comme décrit dans Razafindratsita et al. (2007, J. Allergy Clin. Immunol. 120 : 278-285).

Des groupes de 5 souris ont ensuite été traités par voie sublinguale, deux fois par semaine, pendant deux mois avec :
- 500 μg d'OVA par administration ;
- 10 ou 25 μg d'IgG1κ de rat anti-IgE de souris (clone R35-72 ; BD Biosciences Pharmingen, San Diego, CA, USA) par administration ;
- 25 μg d'IgG1κ de rat non spécifique (isotype contrôle ; eBioscience, San Diego, CA, USA) par administration ou
- du PBS pour le groupe de souris témoin.

Les souris sont alors soumises à une provocation allergénique avec des aérosols d'OVA (1% masse/volume), deux fois en deux jours consécutifs.

Parallèlement, un groupe de trois souris saines non soumises à l'ovalbumine est utilisé comme groupe témoin.

L'anti-IgE utilisé, issu du clone R35-72, est un anticorps de rat de type IgG1κ qui est connu pour sa capacité à se lier aux IgE à la surface des mastocytes et/ou basophiles et d'induire ainsi la libération par ces cellules de médiateurs pro-inflammatoires (Kubo et al. (2003) J. Immunol. 170 : 775-780, Zhou et al. (2007) J. Exp. Med. 204 : 2797-2802).

L'anticorps monoclonal IgG1κ de rat non spécifique est sans spécificité pour les IgE murines.

### Détermination de l'hyperréactivité bronchique.

La mesure de l'hyperréactivité bronchique est effectuée 24 heures après la dernière provocation par une pléthysmographie du corps entier (Buxco Europe Ltd, Winchester, UK) comme décrit par Hamelmann et al. (1997, Am. J. Respir. Crit. Care Med. 156 : 766-775) et la résistance bronchique est estimée par la mesure du PenhH (enhanced pause). L'indice de PenH a été obtenu par détermination du rapport entre les valeurs de PenH mesurées après exposition à une inhalation de métacholine USA et après exposition à du PBS nébulisé.

### Résultats

Afin, de déterminer l'effet d'un anticorps anti-IgE ayant la capacité de lier et de ponter les IgE murines à la surface des mastocytes et des basophiles sur la réponse allergique, des souris ont été sensibilisées à l'ovalbumine puis désensibilisées avec cet anticorps anti-IgE. Ces souris sont comparées avec des souris sensibilisées à l'ovalbumine puis désensibilisées avec la même ovalbumine, ou désensibilisées avec un isotype contrôle, ou non désensibilisées. Toutes ces souris sont comparées à des souris non sensibilisées à l'ovalbumine.

L'hyperréactivité bronchique à la métacholine est accrue chez les souris sensibilisées à l'ovalbumine et traitées au PBS **(****FIG. 1****,** groupe 2), comme le traduit la valeur élevée du paramètre "PenH" en comparaison avec la valeur obtenue pour les souris non sensibilisées à l'ovalbumine **(****FIG. 1****,** groupe 1).

Une amélioration de l'hyperréactivité bronchique est observée chez les souris sensibilisées puis désensibilisées à l'ovalbumine **(****FIG. 1****,** groupe 3).

Une amélioration encore plus importante de l'hyperréactivité bronchique est observée chez les souris sensibilisées à l'ovalbumine puis désensibilisées avec, par ordre croissant d'amélioration :
- l'anti-IgE à raison de 10 μg par administration **(****FIG. 1****,** groupe 6) ;
- l'isotype contrôle à raison de 25 μg par administration **(****FIG. 1****,** groupe 4) ;
- l'anti-IgE à raison de 25 μg par administration **(****FIG. 1****,** groupe 5).

Cet exemple montre de manière très surprenante que l'administration d'un anticorps anti-IgE ayant la capacité de se lier aux IgE à la surface des mastocytes et des basophiles sans dissocier lesdites IgE de leurs récepteurs permet d'induire une diminution très substantielle de l'hyperréactivité bronchique de souris sensibilisées à l'ovalbumine.

Cette diminution est dépendante de la quantité d'anti-IgE administrée et est beaucoup plus efficace qu'une désensibilisation à l'ovalbumine et ceci pour des concentration molaires au moins 60 fois inférieures (sachant que les masses moléculaires de l'ovalbumine et d'une immunoglobuline sont respectivement de 45 kDa (Nisbet et al. (1981) Eur. J. Biochem. 115 : 335-345) et d'environ 150 kDa.

Une partie au moins de l'effet est liée à la spécificité de l'anti-IgE, comme l'indique la différence de la valeur de PenH observée à 25 μg par administration entre l'anti-IgE et l'isotype contrôle, qui est du même ordre que la différence observée entre l'allergène (ovalbumine) et le placebo (PBS). L'effet obtenu avec l'isotype contrôle peut éventuellement être expliqué par l'induction de signaux non spécifiques réduisant la réaction anaphylactique lors de la fixation de cet anticorps sur le récepteur à IgG inhibiteur, le RFcγIIb (Kang et al. (2007) Immune Network 7 : 141-148).

### EXEMPLE 2

Cette étude montre l'effet d'anticorps anti-IgE pontants ou non-pontants ainsi que d'anticorps de type IgG₁ administrés par voie sublinguale en immunothérapie en utilisant un modèle murin *in vivo* d'asthme allergique.

### Matériel et méthodes

### Souris, réactifs et anticorps

Des souris femelles âgées de 6 à 8 semaines ont été obtenues de Charles River (L'Arbresle, France). Le tampon phosphate (PBS) provient d'Invitrogen (Carlsbad, CA). L'ovalbumine (OVA) niveau V avec une faible teneur en endotoxine a été obtenue de Sigma (St. Louis, MO) et a été en outre purifiée sur un gel éliminant les endotoxines (Pierce, Rockford, IL). Les concentrations en endotoxine résiduelle, déterminées par test Endochrome K (R1708K, Charles River, Wilmington, MA), étaient toujours inférieures à 0,1 unité enzymatique (UE)/μg de protéine. Une forme polymérisée de maltodextrine de maïs (polysaccharide capsulaire ou PSC) a été utilisée comme système d'administration d'antigène particulaire muco-adhésif (Baudner et al. (2002) Infect. Immun. 70:4785-4790; Razafindratsita et al. (2007) J. Allergy Clin. Immunol. 120:278-285).

Les anticorps monoclonaux (mAb) cités dans le tableau 1 ont été utilisés comme anticorps purifiés pour une administration sublinguale.

**Tableau 1 : Liste des anticorps utilisés.**

| **Anticorps** | **Antigène** | **Espèce/Isotype** | **Fabricant** |
|---|---|---|---|
| LO-ME-2 | IgE (pontant) | Rat IgG₂ₐ/κ | Invitrogen, ref: 04-7000 |
| Isotype contrôle | IgE | Rat IgG₂ₐ | e-biosciences, ref: 14-4321 |
| R35-92 | IgE (non pontant) | Rat IgG₁/κ | BD Biosciences, ref: 553416 |
| R35-72 | IgE (pontant) | Rat IgG₁/κ | BD Biosciences, ref: 553413 |
| Isotype contrôle | IgE | Rat IgG₁/κ | e-biosciences, ref: 14-4301 |

### Purification des anticorps

Afin d'obtenir tous les anticorps dans un tampon identique, les échantillons d'anti-IgE ont été dialysés contre 10 volumes de PBS en utilisant des membranes de 30 kD (Amicon Ultra-4, Millipore Corp.). Les échantillons désalés ont été en outre filtrés (Millex 0,22 μm, Millipore Corp.) afin d'empêcher toute croissance bactérienne en absence d'azoture de sodium. Enfin, les concentrations protéiques ont été déterminées en utilisant la densité optique à 280 nm (OD280) (Secoman XL, Uvikon) avant et après filtration.

### Immunothérapie sublinguale dans les souris BALB/c

Pour la sensibilisation, les souris ont été immunisées par voie intrapéritonéale (i.p.) aux jours 0 et 14 avec 10 μg d'OVA adsorbés sur 2 mg d'Al(OH)₃, administré dans 100 µl de PBS. Au jour 21, un test de provocation de 20 min par aérosol a été réalisé avec 1% (poids/volume) d'OVA sur 4 jours consécutifs en utilisant un système d'administration d'aérosol (Buxco Europe Ltd, Winchester, UK). Pour l'induction de la tolérance, les anticorps anti-IgE (10 µg et 25 µg par dose) ont été appliqués par voie sublinguale à des groupes de 8 souris, deux fois par semaine pendant 2 mois. Les souris témoins ont été traitées par voie sublinguale avec du PBS ou des anticorps isotypique (IgG₁/κ et IgG₂ₐ). Comme contrôle positif de l'efficacité, des souris ont été traitées par voie sublinguale avec PSC-OVA (500 μg). Des mesures d'hyperréactivité des voies respiratoires (AHR) ont été réalisées par pléthysmographie du corps entier (Puxco Europe Ltd, Winchester, UK) et les résultats ont été exprimés en allongement de la pause *("enhanced pause"* ou Penh). L'index Penh, exprimé comme une augmentation par rapport à la résistance basale des voies respiratoires, a été obtenu en divisant la valeur Penh mesurée après exposition par inhalation à des doses croissantes de métacholine (de 0 à 100 mg) par la valeur Penh mesurée après inhalation de PBS nébulisé, comme décrit dans Razafindratsita *et al.* (2007).

Pour l'analyse des cellules inflammatoires dans les lavages broncho-alvéolaires (BAL), les souris ont été anesthésiées par injection intrapéritonéale de pentobarbital (50 mg/kg de masse corporelle), et les BAL ont été réalisés avec 3 x 400 μl de PBS. Le fluide des BAL a été centrifugé à 800 g pendant 10 min à 4°C. Les culots cellulaires ont été resuspendus dans du PBS, mis à tourner sur des lames de verre par cytocentrifugation, fixés et marqués avec du May/Grünwald Giema (Réactifs RAL, Martillac, France). Les éosinophiles et les macrophages ont été comptés sous microscopie optique en utilisant un grossissement de 200 fois.

### Réponse anticorps

Des échantillons de sang ont été collectés au niveau du sinus rétroorbital afin d'évaluer les niveaux d'anticorps spécifiques d'OVA par ELISA. Les sera ont été collectés après centrifugation à 10000 rpm pendant 10 min. Pour la détection des anticorps IgG₁ et IgG₂ₐ, de l'OVA purifié (0,2 μg) a été étalé pendant la nuit à 4°C sur des plaques ELISA (Nunc, Roskilde, Danemark). Après des étapes de lavage et de saturation, les dilutions de sera de souris (1/100 à 1/12800 pour IgG₁ et 120 à 1/12560 pour IgG₂ₐ) ont été incubées pendant 1 h à 37°C. Les plaques ont été lavées et des IgG₁ de rat biotinylées anti-souris (dilution 1/100, BD Pharmingen, San Jose, CA) ou des anticorps IgG₂ₐ (dilution 1/200, BD Pharmingen) ont été ajoutés pendant 1 h à 37°C. Des anticorps IgG de rat anti-souris conjugués à de la streptavidine-péroxydase (dilution 1/400, BD Pharmingen) ont été utilisés pour la détection, en utilisant des l'orthophénylènediamine (OPD) comme substrat (Sigma Chemicals Aldrich). La réaction a été arrêtée avec de l'HCl 3N et les densités optiques ont été déterminées en utilisant un lecteur de plaques ELISA à 492 nm (Labsystems, Helsinki, Finlande).

Pour la détection des titres en anticorps IgE, des anticorps IgE anti-souris (1 µg/puits, Bethyl Laboratories, Montgomery, Texas) ont été étalés sur des plaques ELISA. Après des étapes de lavage et de saturation, des dilutions des sera de souris (1/10 à 1/320) ont été incubées pendant 1 h à 37°C. De la digoxygénine-OVA a été incubée (à une dilution 1/10) pendant 1 h à 37°C et des fragments Fab d'anticorps de souris anti-digoxygénine conjugués à de la peroxydase de raifort (HRP) (Roche) ont été utilisés pour la détection à une dilution de 1/1000. Un substrat acide 2,2'-azino-bis(3-ethylbenzthiazoline-6 sulfonique) (ABTS) a été ajouté (Roche). Les densités optiques ont été mesurées en utilisant un lecteur de plaques ELISA à 405 nm.

Les titres en anticorps ont été définis comme l'inverse de la dernière dilution pour laquelle la valeur de la densité optique est 2 fois au-dessus du bruit de fond.

Pour déterminer les niveaux d'anticorps spécifiques de l'allergène au niveau des surfaces mucosales, des échantillons de salive ont été collectés afin de réaliser une détermination du niveau d'IgA par ELISA. Brièvement, des microplaques ont été recouvertes d'IgA de chèvre anti-souris (0,1 µg/puits, Bethyl Laboratories), lavées, et des dilutions du surnageant (1/20 à 1/320) ont été incubées pendant 1 h à 37°C, suivi par de la digoxygénine-OVA (dilution 1/100). Les plaques ont été lavées et de des anticorps de lapin anti-digoxygénine conjugués à HRP (Roche) ont été utilisés pour la détection, comme décrit ci-dessus.

### Analyse statistique

Les données ont été comparées en utilisant un test non-paramétrique (Kruskal-Wallis). Les résultats ont été considérés comme statistiquement significatifs à une valeur p inférieure à 0,05.

### Résultats

### Les anti-IgE des clones R35-72 et R35-92 ainsi que les isotypes IgG₁/κ correspondants améliorent l'efficacité clinique chez des souris sensibilisées spécifiquement à OVA et dans l'inflammation pulmonaire.

Comme décrit ci-dessus, les souris sensibilisées avec OVA développent une hyperréactivité des voies respiratoires (AHR) associée à des valeurs Penh élevées détectables par pléthysmographie du corps entier, ainsi que des signes d'inflammation des poumons avec des infiltrations cellulaires. Des anticorps anti-IgE (clones LO-ME-2, R35-72 et R35-92) ont été testés comme candidats pour de l'immunothérapie dans ce modèle murin *in vivo* d'asthme établi. Des anticorps d'isotype correspondant et PSC-OVA ont été utilisés comme contrôles dans ces expériences.

Comme montré en **Fig. 2****,** et comme attendu, les souris saines (*i.e.* non sensibilisées) présentaient des valeurs Penh basses tandis que les souris sensibilisées à OVA traitées par voie sublinguale avec du PBS (placebo) présentaient une forte AHR. Les souris sensibilisées à OVA traitées par voie sublinguale avec PSC-OVA ont été utilisées comme contrôle positif et montraient de faibles valeurs Penh. Un traitement sublingual avec le contrôle isotypique IgG₂ₐ/κ ou avec des anticorps anti-IgE (isotype IgG₂ₐ/κ) du clone LO-ME-2 (10 ou 25 μg) n'a pas eu d'impact sur l'AHR. Au contraire, un traitement sublingual avec des anti-IgE (isotype IgG₁) des clones R35-72 et R35-92 a induit une réduction de l'AHR chez la plupart des animaux par rapport aux souris traitées avec du PBS. De manière surprenante, le contrôle isotypique IgG₁ a induit une réduction similaire de l'AHR, suggérant que des mécanismes immunitaires non spécifiques de l'antigène sont impliqués dans l'induction de la tolérance dans ce modèle.

La diminution de l'AHR observée après traitement avec les anti-IgE correspondant aux clones R35-72 et R35-92 (10 ou 25 μg) et le contrôle isotypique IgG₁ correspondant a été associée à une diminution significative (p<0,05) du nombre d'éosinophiles dans les BAL, comme observé avec le contrôle positif PSC-OVA **(****Fig. 3****).**

### L'administration sublinguale thérapeutique d'anticorps anti-IgE n'altère pas les réponses IgG, IgE ou IgA.

L'immunothérapie sublinguale (SLIT) avec les anticorps anti-IgE du clone LO-ME-2 (isotype IgG₂ₐ/κ) a augmenté les IgG₁ et IgE spécifiques d'OVA seulement à la dose de 10 μg. Dans tous les autres groupes expérimentaux, il n'y avait pas de changement détectable dans les anticorps sériques IgE ou IgG spécifiques d'OVA. Les niveaux d'anticorps salivaires IgA spécifiques d'OVA étaient accrus chez les souris traitées avec PSC-OVA par rapport aux souris ayant reçu du PBS. En revanche, aucun des anticorps anti-IgE ni les anticorps contrôles avec les isotypes correspondants n'ont altéré le niveau d'anticorps IgA spécifiques d'OVA.

Ainsi cette étude montre que des anticorps anti-IgE d'isotype IgG₁/κ des clones R35-72 et R35-92 permettent d'augmenter l'induction de la tolérance. De plus, les anticorps contrôles isotypiques IgG₁/κ promeuvent également l'induction de la tolérance. Au contraire, des anticorps anti-IgE d'isotype IgG₂ₐ/κ du clone LO-ME-2 et les anticorps contrôles isotypiques IgG₂ₐ/κ n'altèrent pas la fonction pulmonaire chez les souris sensibilisées à OVA. Ainsi, ces données suggèrent que l'effet inducteur de tolérance des anticorps anti-IgE des clones R35-72 ou R35-92 pourrait impliquer la région Fc des isotypes IgG₁/κ. Ceci lève la possibilité que de tels anticorps anti-IgE pourraient médier leur effet inducteur de tolérance à la fois en se liant aux IgE liées à FcεRI par leurs régions Fab, et en se liant au récepteur régulateur FcγRIIb, via leur région Fc.

### EXEMPLE 3

Cette étude montre la distribution des récepteurs Fc des IgG dans les tissus linguaux de la souris et la caractérisation de la N-glycosylation des régions Fc des anticorps utilisés dans l'exemple 2.

### Matériel et méthodes

### Immuno-histologie

Pour l'immunohistologie, des tissus provenant de la rate et de la langue ont été prélevés sur des souris naïves et congelés à -80°C. Des sections de tissus (4-6 µm de largeur) ont été coupées en série, séchées à l'air pendant au moins 30 min, fixées dans l'acétone pendant 1-2 min, et incubées pendant 10 min dans 3% de peroxyde d'hydrogène (Sigma) pour bloquer l'activité peroxydase endogène. Après lavage dans du tampon Tris (TBS : 0,05 M Tris, 0,15 M NaCl, pH 7,4), les anticorps primaires *i.e.* anti-CD16/32 (clone 2.4G2, BD Biosciences) ou anti-SIGN-R1 (clone ER-TRP9, Abcam) (dilution 1/100 en TBS) ont été ajoutés aux échantillons et incubés pendant 1 h à température ambiante. Les sections de tissu ont été lavées dans du TBS et incubées avec des anticorps secondaires IgG de lapin anti-chèvre biotinylés (Sigma, 1/400) pendant 30 min avant d'ajouter de la peroxydase de raifort biotine-streptavidine (SA-HRP, Sigma). Après 30 min, les échantillons ont été lavés et le marquage spécifique a été visualisé en utilisant la diaminobenzidine (DAB, Sigma) comme substrat. Des sections de tissus réalisées en l'absence de l'anticorps primaire ont été incluses comme contrôles négatifs.

### Analyse de la N-glycosylation des anticorps en utilisant LC-ESIMS

Les anticorps IgG de rat anti-IgE (R35-72 et R35-92 de BD Biosciences, eBRG1 et eBR2 d'eBiosciences, LO-ME2 d'Invitrogen) ont été dénaturés (urée 6 M) et réduits (75 mM DTT, 50°C, 15 min) avant analyse par LC-ESIMS. L'analyse de chaînes lourdes réduites par ESIMS a été réalisée après séparation chromatographique des chaînes légères et des chaînes lourdes. Les IgG ont été également soumises à la LC-ESIMS après déglycosylation (traitement PNGase F, Glycoprofile II, Sigma) ou élimination enzymatique de l'acide sialique (sialidase Au, QA Bio) selon les instructions du fabricant. Brièvement, environ 2 µg d'anticorps réduit ont été injectés sur une Acquity C₄, 10 cm x 2,1 mm, 1,7 µm (BEH300, Waters) thermostatée à 80°C et connectée à un système RS-HPLC (Dionex). Un gradient de CH₃CN (avec 0,1% v/v d'acide formique) a été réalisé avec une vitesse de flux de 400 µl/min pour assurer une détection UV correcte à 210 nm. Un spectromètre de masse Qq-TOF (Maxis, Bruker) a été connecté au RS-HPLC pour une mesure de masse correcte et opérait en mode d'ionisation positive. La déconvolution du spectre de masse a été réalisée en utilisant l'algorithme MaxEnt (Waters Corp.) en utilisant les paramètres suivants : 45000-55000 Da, auto-espacement des points de données, résolution 40000. Ainsi, les masses des chaînes lourdes intactes, désialylées et déglycosylées ont été obtenues de manière à identifier les profils de glycosylation.

### Résultats

### Identification des cellules portant les récepteurs Fc des IgG dans les tissus de la langue des souris BALB/c naïves et sensibilisées à OVA

Afin de déterminer la distribution tissulaire du récepteur Fc des IgG, les inventeurs ont analysé les tissus linguaux de souris BALB/c naïves et sensibilisées à OVA par immunohistologie en utilisant des anticorps spécifiques, *i.e.* CD16/CD32 et SIGN-R1. CD16/CD32 a été détecté à l'interface mucosal/sous-mucosal à la fois dans des sites tissulaires ventraux et dorsaux de la langue ainsi que dans la zone musculaire, à la fois chez les souris naïves et les souris sensibilisées à OVA. En revanche, SIGN-R1 a seulement été détecté dans le tissu musculaire (à la fois chez les souris naïves et sensibilisées à OVA), loin du site d'administration sublinguale.

### Analyse de la N-glycosylation des anticorps par LC-ESIMS

Les IgG ont été dénaturées et réduites avant analyse par LC-ESIMS des chaînes lourdes intactes, désialylées et déglycosylées, afin d'identifier leurs profils de glycosylation respectifs.

Les différentes structures oligosaccharidiques ont été déterminées par LC-ESIMS, à la fois avant et après digestions enzymatiques (*i.e.* déglycosylation et élimination de l'acide sialique). L'anticorps R35-92 présentait une hétérogénéité structurelle des glycoformes de chaînes lources, avec l'addition successive de différents monosaccharides à l'oligosaccharide lié en N. Le spectre de masse de l'anticorps R35-92 déglycosylé montre la masse (49075 Da) du polypeptide de la chaîne lourde tandis que le spectre de masse de la chaîne lourde native montre une augmentation de masse de 1298 Da correspondant à l'addition d'une structure non-fucosylée, non-galactosylée, non-sialylée, biantennaire (masse observée 50373 Da). Des glycanes biantennaires sialylés, galactosylés et fucosylés ont également été mis en évidence. Le traitement à la sialidase a confirmé la présence de structures biantennaires fucosylées à la fois mono et bisialylées. Des expériences similaires ont été réalisées sur chanque anticorps. Les résultats sont résumés dans le tableau 2 ci-dessous.

**Tableau 2 : Résumé de la caractérisation de la N-glycosylation des chaînes lourdes**

| **Clone** | **Isotype** | **Activité** | **Sialylation** | **Fucosylation** |
|---|---|---|---|---|
| R35-72 | IgG₁ | + | ND | 40% |
| R35-92 | IgG₁ | + | 30% | 50% |
| eBRG1 | IgG₁ | + | ND | ND |
| eBRG2 | IgG₂a | - | ND | 100% |
| LO-ME* | IgG₂a | - | ND | 50% |

| | | | | |
|---|---|---|---|---|
| ND: non détecté *: l'anticorps LO-ME présente un profil de spectre de masse inhabituel | | | | |

Selon la RS-HPLC suivie de l'analyse ESIMS de haute résolution et de haute précision, tous les anticorps monoclonaux IgG de rat anti-IgE testés (R35-72, R35-92, eBRG1, eBRG2 et LO-ME2) présentent des chaînes lourdes N-glycosylées. Des structures oligosaccharidiques régulières, dont la majorité est biantennaire non-galactosylée, sont observées. La glycosylation diffère entre les anticorps puisqu'une sialylation significative (environ 30%) a uniquement été observée dans l'échantillon R35-92. De plus, l'absence de fucose coeur (échantillon eBRG1) et une fucosylation partielle ou totale (échantillons R35-92 et eBRG2 respectivement) ont également été mise en évidence. Ainsi, ni la sialylation ni la fucosylation ne joue de rôle critique en ce qui concerne l'activité d'induction de la tolérance.

### EXEMPLE 4

Cette étude présente l'identification des récepteurs de la partie Fc des IgG₁ au niveau des tissus buccaux humains.

Les anticorps anti-CD32 (clone AT10, ref ab41899, Abcam) reconnaissent la famille des récepteurs inhibiteurs CD32.

La présence de cellules exprimant CD32 est détectée par immuno-histochimie au niveau de la jonction muqueuse/sous-muqueuse du tissu de gencives humaines.

Les inventeurs ont observé un marquage d'intensité modérée à marquée de cellules immunitaires en quantité très importante dans le chorion papillaire (face buccale).

Les anticorps anti-CD16 (clone 2H7, ref ab74512, Abcam) reconnaissent la famille des récepteurs activateurs CD16.

La présence de cellules exprimant CD16 est détectée par immuno-histochimie au niveau de la jonction muqueuse/sous-muqueuse du tissu de gencives humaines.

Les inventeurs ont observé un marquage d'intensité modérée de cellules immunitaires en faible quantité dans le chorion papillaire (face buccale).

Ainsi, les inventeurs ont montré qu'au niveau des gencives humaines, la balance entre les récepteurs activateurs CD16 et récepteurs CD32 (qui comprennent des récepteurs inhibiteurs) était en faveur de ces derniers. Ils sont détectés en plus forte quantité au niveau du chorion papillaire. Par conséquent, la présence d'un plus grand nombre de récepteurs CD32 est en faveur de l'utilisation de ligands de CD32, et en particulier d'immunoglobuline IgG₁, par voie mucosale, en particulier par voie sublinguale, chez l'homme pour traiter les maladies et manifestations inflammatoires.

## Revendications

1. Anticorps anti-IgE de type IgG1 ayant la capacité de lier les IgE fixées aux récepteurs à la surface d'un mastocyte ou basophile sans dissocier lesdites IgE de leurs récepteurs et de ponter des IgE fixées aux récepteurs à la surface d'un mastocyte ou basophile, pour une utilisation dans le traitement d'une hypersensibilité de type 1, l'anticorps anti-IgE étant administré par voie mucosale.

2. Anticorps anti-IgE pour l'utilisation selon la revendication 1, l'anticorps anti-IgE reconnaissant une partie de l'IgE non impliquée dans la fixation de l'IgE à son récepteur sur le mastocyte ou basophile.

3. Anticorps anti-IgE pour l'utilisation selon l'une quelconque des revendications 1 à 2, l'anticorps anti-IgE étant un anticorps monoclonal, un anticorps chimérique, un anticorps humain ou humanisé.

4. Anticorps anti-IgE pour l'utilisation selon l'une quelconque des revendications 1 à 3, l'anticorps anti-IgE étant administré par voie sublinguale.

5. Anticorps anti-IgE pour l'utilisation selon l'une quelconque des revendications 1 à 4, l'hypersensibilité de type 1 se manifestant par une anaphylaxie systémique, une anaphylaxie localisée, une rhinite allergique, de l'asthme, une dermatite atopique, une conjonctivite, de l'eczéma, une mastocytose induite par un choc anaphylactique, une manifestation allergique associée à une parasitose.

6. Composition pharmaceutique pour une utilisation dans le traitement d'une hypersensibilité de type 1 par voie mucosale, comprenant un anticorps anti-IgE tel que défini dans l'une quelconque des revendications 1 à 3.

7. Composition pharmaceutique pour l'utilisation selon la revendication 6 comprenant en outre un allergène ou un antigène.

8. Composition pharmaceutique pour l'utilisation selon la revendication 6 ou 7, la composition étant administrée par voie sublinguale.

## Patentansprüche

1. Anti-IgE-Antikörper des Typs IgGi mit der Fähigkeit, die an den Rezeptoren auf der Oberfläche eines Mastozyten oder Basophils fixierten IgE zu binden, ohne die IgE von ihren Rezeptoren zu trennen, und die auf der Oberfläche eines Mastozyten oder Basophils fixierten IgE zu überbrücken, für eine Verwendung bei der Behandlung einer Hypersensibilität des Typs 1, wobei der Anti-IgE-Antikörper über die Schleimhaut verabreicht wird.

2. Anti-IgE-Antikörper für die Verwendung nach Anspruch 1, wobei der Anti-IgE-Antikörper einen Teil von IgE erkennt, der nicht an der Fixierung des IgE an seinem Rezeptor auf dem Mastozyten oder Basophil beteiligt ist.

3. Anti-IgE-Antikörper für die Verwendung nach einem der Ansprüche 1 bis 2, wobei der Anti-IgE-Antikörper ein monoklonaler Antikörper, ein chimärischer Antikörper, ein humaner oder humanisierte Antikörper ist.

4. Anti-IgE-Antikörper für die Verwendung nach einem der Ansprüche 1 bis 3, wobei der Anti-IgE-Antikörper sublingual verabreicht wird.

5. Anti-IgE-Antikörper für die Verwendung nach einem der Ansprüche 1 bis 4, wobei sich die Hypersensibilität Typ 1 durch eine systemische Anaphylaxie, eine lokale Anaphylaxie, eine allergische Rhinitis, Asthma, eine atopische Dermatitis, eine Bindehautentzündung, Ekzeme, eine durch einen anaphylaktischen Schock induzierte Mastozytose, eine einer Parasitose zugeordnete allergische Manifestation manifestiert.

6. Pharmazeutische Zusammensetzung für eine Verwendung bei der Behandlung einer Hypersensibilität Typ 1 über die Schleimhaut, umfassend einen Anti-IgE-Antikörper wie in einem der Ansprüche 1 bis 3 definiert.

7. Pharmazeutische Zusammensetzung zur Verwendung nach Anspruch 6, umfassend ferner ein Allergen oder ein Antigen.

8. Pharmazeutische Zusammensetzung zur Verwendung nach Anspruch 6 oder 7, wobei die Zusammensetzung auf sublingualem Weg verabreicht wird.

## Claims

1. Anti-IgE antibody of IgG₁ type having the capacity of binding the IgEs fixed to the receptors on the surface of a mastocyte or basophile without dissociating said IgEs from their receptors and of bridging IgEs fixed to the receptors on the surface of a mastocyte or basophile, for use in the treatment of a type 1 hypersensitivity, the anti-IgE antibody being administered via mucosal route.

2. The anti-IgE antibody for use according to claim 1, the anti-IgE antibody recognizing a part of the IgE not involved in the fixing of the IgE to its receptor on the mastocyte or basophile.

3. The anti-IgE antibody for use according to any of claims 1 to 2, the anti-IgE antibody being a monoclonal antibody, a chimeric antibody, a human antibody or humanized.

4. The anti-IgE antibody for use according to any of claims 1 to 3, the anti-IgE antibody being administered via sublingual route.

5. The anti-IgE antibody for use according to any of claims 1 to 4, wherein the sign of the type 1 hypersensitivity is systemic anaphylaxis, localized anaphylaxis, allergic rhinitis, asthma, atopical dermatitis, conjunctivitis, eczema, mastocytosis induced by anaphylactic shock, an allergic sign associated with parasitosis.

6. A pharmaceutical composition for use in the treatment of a type 1 hypersensitivity via mucosal route, comprising an anti-IgE antibody such as defined in any of claims 1 to 3.

7. The pharmaceutical composition for use according to claim 6 further comprising an allergen or an antigen.

8. The pharmaceutical composition for use according to claim 6 or 7, the composition being administrated via sublingual route.
